# EUROPEAN PATENT APPLICATION

(11) **EP 4 122 917 A1**
(43) Date of publication of application: **25.01.2023**
(21) Application number: 21382679.5
(22) Date of filing: 23.07.2021
(51) Int. Cl.: C07D 209/30, A61P 31/12, A61K 31/404

(54) **C2-THIOETHER TRYPTOPHAN TRIMERS AND TETRAMERS AND USE THEREOF**

(71) Applicant: Consejo Superior de Investigaciones Científicas (CSIC), 28006 Madrid (ES); Universitat de Valéncia, 46010 Valencia (ES)
(72) Inventor: SAN-FÉLIX GARCÍA, Ana Rosa, 28006 Madrid (ES); PÉREZ PÉREZ, María Jesús, 28006 Madrid (ES); QUESADA DEL SOL, Ernesto, 28006 Madrid (ES); GARGANTILLA LÓPEZ, Marta, 28006 Madrid (ES); MARTÍNEZ GUALDA, Belén, 28006 Madrid (ES); GELLER, Ron, 46980 Paterna (Valencia) (ES); FRANCÉS GÓMEZ, Clara, 46980 Paterna (Valencia) (ES)
(74) Representative: Pons

(57) **Abstract**

The present invention relates to C2-thioether tryptophan (Trp) trimers and tetramers in which differently substituted thio aryl rings have been introduced at the C2 position of the indole ring of each Trp moiety. The invention also refers to their therapeutic use for the treatment or prevention of betacoronoavirus infections, mainly against SARS-CoV-2 infection.

## Description

The present invention relates to C2-thioether tryptophan (Trp) trimers and tetramers in which differently substituted thio aryl rings have been introduced at the C2 position of the indole ring of each Trp moiety. The invention also refers to their procedure of synthesis and to their therapeutic use, mainly for the treatment of betacoronoavirus infections.

Therefore, the present invention belongs to the field of medicine.

### BACKGROUND ART

Human coronaviruses cause respiratory tract infections that go from a mild disease (in case of HCoV-NL63, HCoV-229E, HCoV-OC43 and HCoV-HKU1) to very severe syndromes (the case of SARS-CoV, MERS and SARS-CoV-2).

Since the end of 2019, SARS-CoV-2 has been expanding worldwide causing the COVID pandemia. Coronaviruses (CoVs) are enveloped viruses with a single stranded positive-sense RNA genome. Spike (S) proteins protrude from the viral envelope and constitute the main target of neutralizing antibodies. These S proteins mediate host cell entry, where various cellular receptors have been implicated, followed by membrane fusion to facilitate virus entry.

Coronaviruses comprise a group of positive strand RNA viruses with large genomes. The virions of coronaviruses are enveloped and decorated with several glycoproteins. Of these, the Spike protein (S) is the principal mediator of receptor binding and fusion with the host cell. Hence, it is a key mediator of viral tropism and pathogenicity. Overall, coronaviruses are classified into four genera, termed alpha, beta, gamma, and delta. The betacoronaviruses include 3 human pathogens, Middle East respiratory syndrome coronavirus (MERS-CoV), and two related viruses termed severe acute respiratory syndrome coronavirus (SARS-CoV) and SARS-CoV-2. The spike protein of these three coronaviruses encodes similar motifs required for carrying out entry and cell fusion, including a short cytoplasmic tail, a transmembrane domain, and an extracellular region. The latter is divided into two subdomains: S1, which encodes the receptor binding region (N-terminal domain and receptor binding domain), and S2, which encodes the determinants for membrane fusion (the fusion peptide and two heptad repeat sequences) (Verma, J., Subbarao, N. A. Arch Virol 166, 697-714 (2021)). The receptor for both SARS-CoV and SARS-CoV-2 is angiotensin-converting enzyme 2 (hACE2) (Li, W., Moore, M., Vasilieva, N. et al. Nature 426, 450-454 (2003) and J. Lang, J. Ge, J. Yu et al. Nature, 581, 125-220 (2020)), while MERS-CoV binds dipeptidyl peptidase-4 (Raj, V., Mou, H., Smits, S. et al. Nature 495, 251-254 (2013)). Despite using two different receptors, structural studies have shown these coronaviruses bind their cognate receptor in similar manner (Verma, J., Subbarao, N. Arch Virol 166, 697-714 (2021)). The high functional similarity of betacornavirus spike proteins suggests that these could be inhibited by similar antiviral compounds.

The main goal of this invention is the development of new antivirals useful to inhibit virus entry through inhibition of the interaction of the viral spike (S) protein with the cellular host receptor(s). These compounds can be effective to protect cells from being infected by the virus and might be useful for the treatment of SAR-CoV-2 infections. Moreover, they could also be useful to prevent viral infection.

### SUMMARY OF THE INVENTION

The inventors of the present invention have identified a group of compounds (tryptophan trimers and tetramers) that show antiviral activity. These compounds protect cells from being infected by betacoronavirus, such as SARS-CoV-2.

Then, a first aspect of the present invention relates to a compound of formula (I) or (II):# 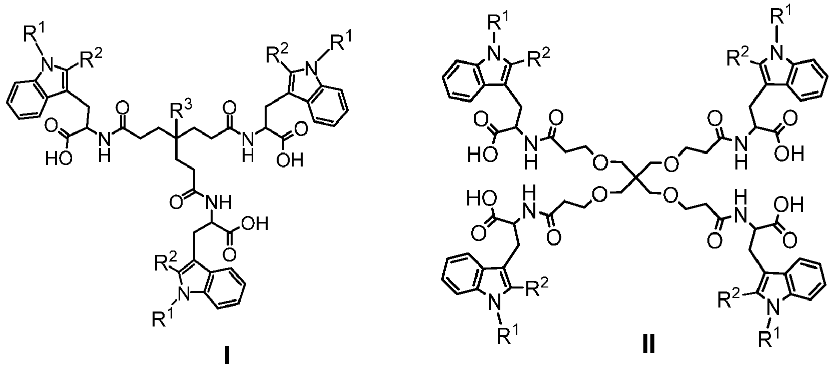 a pharmaceutically acceptable salt, isomers or a solvate thereof, wherein:
R¹ is -H, -CH₃ or -(CH₂)ₙPh, wherein n is 1 or 2 and Ph is a phenyl group which is unsubstituted or substituted by at least one radical selected from the list consisting of: halogen, NO2 and CF₃,
R² is -SPh, wherein the Ph is a phenyl group which is unsubstituted or substituted by at least one radical selected from the list consisting of: halogen, CF₃, NO₂, -CN, -NH-SO₂CH₃, -SO₂NH₂, -COCH₃ and -SO₃H,
R³ is -CH₃, -NH₂ or-NHCOWwherein W is -(CH₂)ₚCH₃, -CH₂(OCH_{2C}H₂)ₚOCH₃ , being p an integer between 2 and 10, or -CH(Y)NHZ, wherein
   Z is -H, -COCH₃ or -CO(CH₂)_{q}CH₃, being q an integer between 4 and 10 and
   Y is a group selected from the list consisting of: H, C₁-C₄ alkyl group unsubstituted or substituted by one radical selected from: phenyl, hydroxyphenyl, -COOH, - CONH₂, -SH, -SCH₃, -OH, -NH₂, 1H-imidazol-4-yl, -CH₂-(1H-indol-3-yl) and 2-pyrrolidinyl.

The term "halogen" refers to fluoride, chloride, bromide or iodine.

The term "C₁-C₄ alkyl group" in the present invention refers to linear or branched saturated hydrocarbon chain that have 1 to 4 carbon atoms, for example, methyl, ethyl, *n*-propyl, *i*-propyl, *n*-butyl, *sec*-butyl, *tert*-butyl.

In a preferred embodiment, R¹ is -H.

In a preferred embodiment, R² is -SPh wherein Ph is a phenyl group which is substituted by one or more radical selected from the list consisting of: -NO₂ and - COCH₃.

In another preferred embodiment, R² is -SPh wherein Ph is a phenyl group which is unsubstituted or substituted by one radical selected from the list consisting of: halogen, -CF₃, -NO₂, -CN, -NH-SO₂CH₃, -SO₂NH₂, -COCH₃, -SO₃H, more preferably -NO₂ or -COCH₃. Even more preferably, R² is -SPh wherein Ph is a phenyl group which is substituted by one radical selected from the list consisting of: halogen, -CF₃, -NO₂, -CN, -NH-SO₂CH₃, -SO₂NH₂, -COCH₃, -SO₃H, more preferably -NO₂ or - COCH₃, in position para with respect to the S atom bonded to the Ph group.

In a preferred embodiment, R³, when present (in the trimer of formula (I)), is -NHCOW, more preferably, R³ is -NHCOCH(Y)NHZ.

In a preferred embodiment, R³ is -NHCOCH(Y)NHZ wherein Y is a group selected from the list consisting of: -H, -CH₃, -CH₂Ph, -CH₂Ph-4-OH, -CH₂COOH, -CH₂CH₂COOH, -CH₂CONH₂, -CH₂CH₂CONH₂, -CH₂SH, -CH₂OH, -CH₂-(1H-imidazol-4-yl), -CH₂CH(CH₃)₂, -CH(CH₃)CH₂CH₃, -(CH₂)₄NH₂, -CH(CH₃)OH, -CH₂-(1H-indol-3-yl), -(CH₂)₂SCH₃, -CH₂-(2-pyrrolidinyl) and -CH₂CH(CH₃)₂. More preferably, Y is H.

In a preferred embodiment, R³, when present, is -NHCOCH₂NH₂ (both Y and Z are H) or - NHCOCH₂NHCO(CH₂)_{q}CH₃ (in this case, Y is H and Z= CO(CH₂)_{q}CH₃), being q as defined above (an integer between 4 and 10).

In a preferred embodiment, the compound of formula (I) or (II) is selected from the next ones: 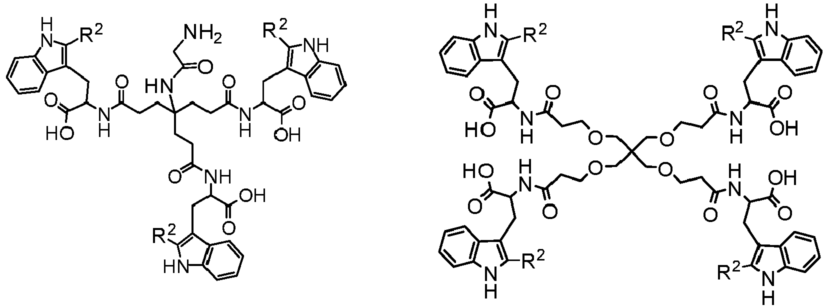 wherein R² is: 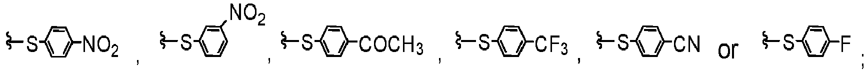 more preferably R² is:

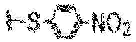

In a preferred embodiment, the compound of formula (I) is a compound of formula: 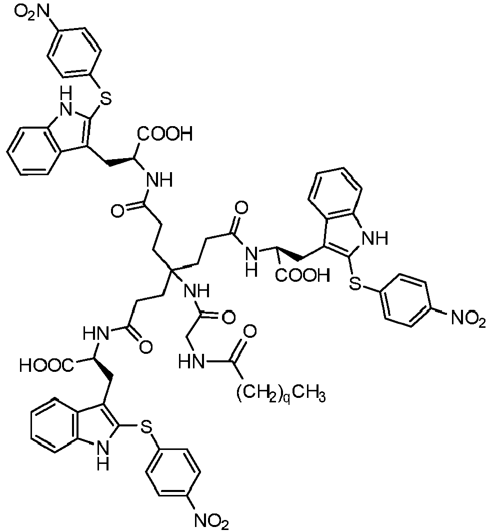 wherein q is 4, 6, 8 or 10, preferably q is 6.

Unless otherwise stated, the compounds of formula (I) or (II) are intended to include compounds that differ only in the presence of one or more isotopically enriched atoms. For example, compounds having the present structures except for the substitution of a hydrogen atom for a deuterium atom or a tritium atom, or the substitution of a carbon atom for a carbon atom enriched in ¹³C or ¹⁴C or a nitrogen atom enriched in ¹⁵N fall within the scope of this invention.

It must be understood that compounds of formula (I) or (II) encompasses all the isomers of said compounds, i.e. all the geometric, tautomeric and optical forms, and their mixtures (for example, racemic mixtures). When there are more chiral centres in the compounds of formula (I), the present invention includes, within its scope, all the possible diastereomers, including their mixtures. The different isomeric forms may be separated or resolved therebetween using conventional methods or any given isomer can be obtained using conventional synthetic methods or by means of stereospecific, stereoselective or asymmetric synthesis.

The term "pharmaceutically acceptable salts or solvates thereof" relates to salts or solvates which, on being administered to the recipient, are capable of providing a compound such as that described herein. The preparation of salts and derivatives can be carried out by methods known in the state of the art. Preferably, "pharmaceutically acceptable" relates to molecular entities and compositions that are physiologically tolerable and do not typically produce an allergic reaction or a similar unfavourable reaction, such as gastric upset, dizziness and similar side effects, when administered to a human. Preferably, the term "pharmaceutically acceptable" means approved by a regulatory agency of a federal or state government or collected in the US Pharmacopoeia or other generally recognised pharmacopeia for use in animals and, more particularly, in humans.

The compounds of formula (I) or (II) may be in crystalline form, either as free compounds or as solvates (e.g.: hydrates), and it is understood that both forms fall within the scope of the present invention. Solvation methods are generally known in the state of the art. Suitable solvates are pharmaceutically acceptable solvates. In a particular embodiment, the solvate is a hydrate.

A second aspect of the invention refers to a compound of formula (I) or (II), as defined in the first aspect of the invention, for use as a medicament. Preferably, the medicament is an antiviral agent.

A third aspect of the invention refers to a compound of formula (I) or (II), as defined in the first aspect of the invention, for use in the treatment and/or prevention of betacoronavirus infection.

Preferably, the betacoronavirus is selected form SARS-CoV, MERS and SARS-CoV-2, and, even more preferably, the betacoronavirus is SARS-CoV-2.

Other aspect of the invention refers to the use of compound of formula (I) or (II), as defined in the first aspect of the invention, for the preparation of a medicament, preferably for the treatment and/or prevention of betacoronavirus infection, more preferably SARS-CoV, MERS or SARS-CoV-2 infection.

Other aspect of the invention refers to a method for the treatment and/or prevention of betacoronavirus infection, preferably SARS-CoV, MERS or SARS-CoV-2 infection, in a subject, comprising the administration of a therapeutically effective amount of a compound of formula (I) or (II), as defined in the first aspect of the invention.

The term "treatment or prevention" as used herein, unless otherwise indicated, relates to reversing, alleviating and inhibiting the progress of, or preventing the disorder or condition to which it applies in such terms, one or more symptoms of such disorder or condition.

Other aspect of the invention refers to a pharmaceutical composition comprising the compound of formula (I) or (II), as described above, a pharmaceutically acceptable salt, isomers or a solvate thereof.

The pharmaceutical composition preferably includes at least one excipient, adjuvant and/or a pharmaceutically acceptable carrier.

The term "carrier" relates to a dilute, adjuvant or excipient with which the main ingredient is administered. Such pharmaceutical carriers may be sterile liquids, such as water and oils, including those derived from oil, animal, vegetable or synthetic, such as peanut oil, soybean oil, mineral oil, sesame seed oil and similar. Water or aqueous saline solutions and aqueous dextrose and glycerol solutions are preferably used as carriers, particularly for injectable solutions. Preferably, the carriers of the invention are approved by the regulatory agency of a state government or a federal government, or are enumerated in the United States Pharmacopoeia, in the European Pharmacopoeia or other pharmacopoeia recognised in general for use in animals and, more particularly, in humans.

The pharmaceutical composition comprises a therapeutically effective amount of the compound of formula (I) or (II), the pharmaceutically acceptable isomers, salts or solvates thereof that must be administered (also referred to herein as therapeutically amount effective thereof).

The therapeutically amount effective of the compound of formula (I) or (II), in addition to their dosage for treating a pathological condition with said compound, will depend on a number of factors, including age, the condition of the patient, the severity of the disease, administration route and frequency, the modular compound to be used, etc.

The pharmaceutical compositions of this invention can be used on their own or jointly with other drugs to provide combined therapy. The other drugs can form part of the same pharmaceutical composition or be provided as a separate pharmaceutical composition, to be administered simultaneously or at different intervals. Examples of pharmaceutical compositions include any solid composition (tablets, pills, capsules, granules, etc.) or liquid composition (solutions, suspensions or emulsions) for oral, topical or parenteral administration.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples are provided by way of illustration and are not intended to be limiting of the present invention.

### Examples

### Example 1: Synthesis of trimers of formula (I)

### 1.1 Synthesis of trimers of formula (I) wherein R¹ is -H, R² is -SPh (wherein the Ph is substituted by one radical) and R³ is: -NHCOCH₂NH₂ in said formula

For the synthesis of trimers of formula (I) a novel chemical approach consisting of several sequential steps was followed (Schemes 1 and 2). The synthesis started with the commercially available OMe-protected Trp (H-TrpOMe-HCl) **1** whose amino group was protected with the fluorenylmethyl oxycarbonyl group (Fmoc) to afford intermediate **2** (as described in: Richard, D. J.et al. Proc Natl Acad Sci U S A 2004, 101 (33), 11971-11976) in quantitative yield (Scheme 1). Next, thioarylation on the C2 position of the indole moiety with the corresponding phenyl disulfide in the presence of iodine at 60 °C afforded the 2-thioether tryptophan intermediates **3a-f** (55-83% yield). Fmoc deprotection of **3a-f,** using piperidine, afforded intermediates **4a-f** (68-89% yield). Each of these key intermediates has an unmasked amino group ideal for further attachment to a central scaffold. Coupling of these key intermediates with the three carboxylate groups of the Fmoc protected glycine scaffold **5** (described in: Weissenborn, M. J. et al. Chem. Commun. 2012, 48 (37), 4444-4446) in the presence of hexafluorophosphate azabenzotriazole tetramethyl uronium (HATU), as coupling reagent, and *N,N*-diisopropylethylamine (DIPEA), as base, gave trimers **6a-f** (52-76%) (Scheme 2). The subsequent treatment of **6a-f** with LiOH·H₂O, followed by acidification at pH = 2, resulted in saponification of the protecting ester moieties with concomitant removal of the Fmoc protecting group to give the target deprotected trimers **7a-f** (from 40% to 94% yields) (Scheme 2).

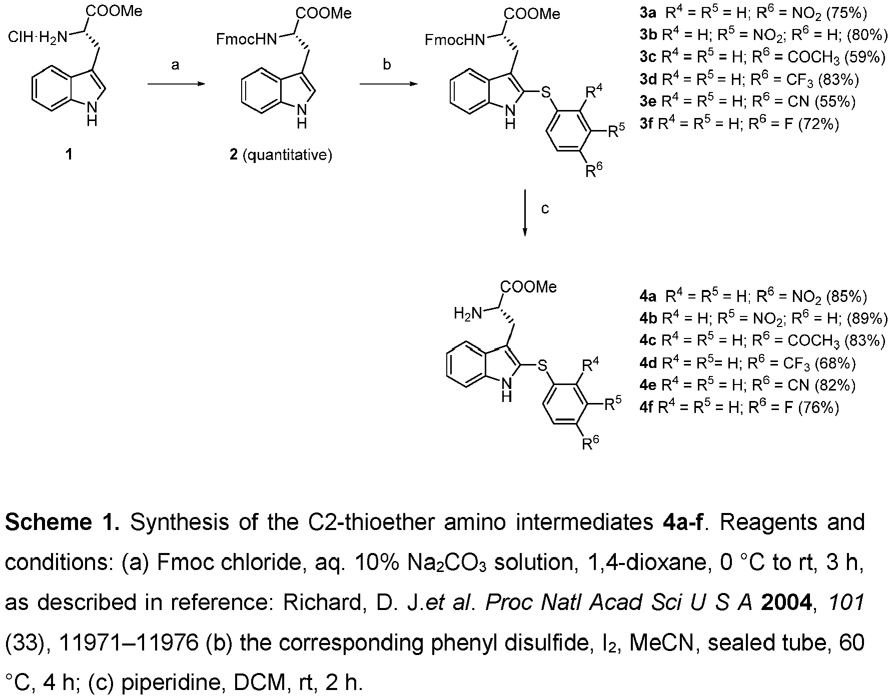

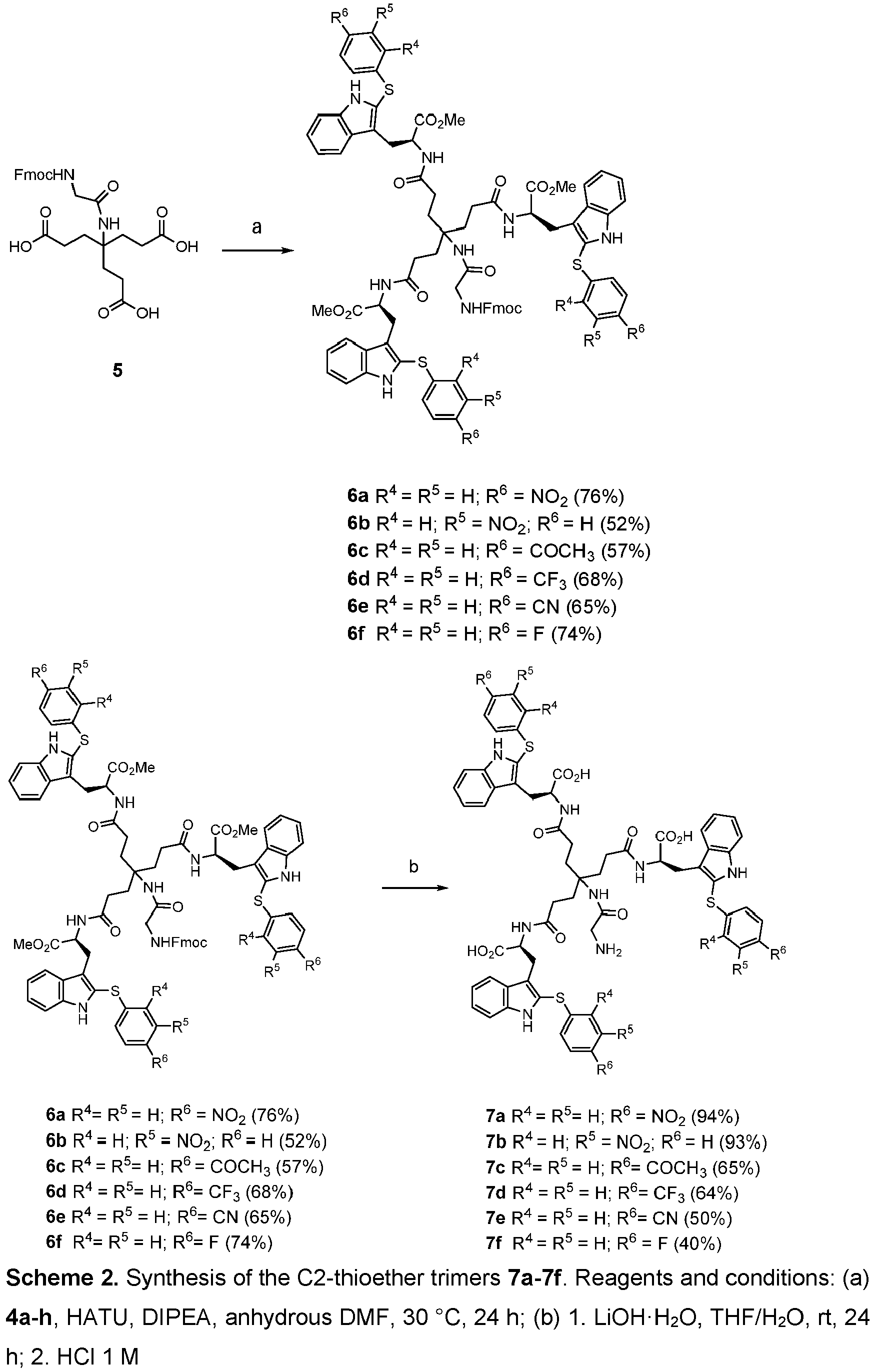

The disulfides used are commercially available (Merck), except 4-trifluoromethylphenyldisulfide synthesized as described in Zheng, Y.; Qing, F.-L.; Huang, Y.; Xu, X.-H. Tunable and Practical Synthesis of Thiosulfonates and Disulfides from Sulfonyl Chlorides in the Presence of Tetrabutylammonium Iodide. Adv. Synth. Catal. 2016, 358 (21), 3477-3481) and 4-acetylphenyldisulfide, whose synthesis is described below.

### Synthesis of 4-acetylphenyldisulfide

Following the procedure described in Zheng, Y. Adv. Synth. Catal. 2016, 358 (21), 3477-3481, to a solution containing 4-acetylbenzenesulfonyl chloride (1.0 g, 4.58 mmol) in anhydrous DMF (13 mL), a solution of tetrabutylammonium iodide (TBAI) (5.06 g, 13.92 mmol) in anhydrous DMF (13 mL) was added dropwise. The resulting solution was stirred at rt for 24 hours. Then, it was diluted with DCM (20 mL) and quenched with an aqueous solution of Na₂S₂O₃ (10 mL). The organic layer was washed with a saturated solution of NaHCO₃ (10 mL), dried over Na₂SO₄, filtered and evaporated to dryness. The residue was purified by flash chromatography (DCM/hexane, 1:1) to yield 381 mg (22%) of the title compound as orange oil. Spectroscopic characterization of this compound was consistent with that described in the literature (Yang, Z.; Shi, Y.; Zhan, Z.; Zhang, H.; Xing, H.; Lu, R.; Zhang, Y.; Guan, M.; Wu, Y. Sustainable Electrocatalytic Oxidant-Free Syntheses of Thiosulfonates from Thiols. ChemElectroChem 2018, 5 (23), 3619-3623).

### General procedure for the sulfenylation reaction (intermediates 3a-f).

Fmoc tryptophan methyl ester 2 (Richard, D. J.; Schiavi, B.; Joullié, M. M. Synthetic Studies of Roquefortine C: Synthesis of Isoroquefortine C and a Heterocycle. Proc. Natl. Acad. Sci. 2004, 101 (33), 11971-11976) (1.0 mmol), the corresponding aromatic disulfide (0.7-1.2 mmol) and l₂ (0.6 mmol) were placed in a sealed tube and dissolved in acetonitrile (4 mL). The resulting mixture was heated at 60 °C for 3-6 hours. Then it was diluted with ethyl acetate (20 mL) and washed with an aqueous solution of NaHSO₃ (10 mL). The organic layer was dried over Na₂SO₄, filtered and evaporated to dryness and the residue was purified by flash chromatography.

**Methyl (S)-2-((((9H-fluoren-9-yl)methoxy)carbonyl)amino)-3-(2-((4-nitrophenyl)thio)-1H-indol-3-yl)propanoate (3a).** Following the general procedure for sulfenylation, Fmoc tryptophan methyl ester **2** (800 mg, 1.82 mmol), commercially available 4-nitrophenyldisulfide (467 mg, 1.51 mmol) and l₂ (230 mg, 0.91 mmol) in acetonitrile (6.0 mL) reacted for 3 hours. After workup, the crude product was subjected to column chromatography (DCM/ethyl acetate, 60:1) to yield 672 mg (75%) of **3a** as a yellow solid. Mp 100-102 °C. MS (ES, positive mode): m/z 594 (M+H).+ ¹H NMR (400 MHz, DMSO-*d₆*) *δ*: 3.19 (dd, *J* = 14.2, 8.3 Hz, 1H, NHCHCH₂), 3.28 (m, 1H, NHCHCH₂), 3.49 (s, 3H, COOCH₃), 4.09 - 4.20 (m, 3H, COOCH₂CH), 4.27 (td, *J* = 8.1, 6.6 Hz, 1H, NHCHCH₂), 7.08 (td, *J* = 8.0, 7.0, 1.0 Hz, 1H, Ar), 7.19 (d, *J* = 8.9 Hz, 2H, Ar), 7.23 (m, 1H, Ar), 7.28 (dd, *J* = 7.4, 1.2 Hz, 1H, Ar), 7.31 (dt, *J* = 7.5, 1.2 Hz, 1H, Ar), 7.39 (m, 3H, Ar), 7.62 (dd, 2H, Ar), 7.70 (d, *J* = 8.0 Hz, 1H, Ar), 7.88 (dd, *J =* 7.6, 1.1 Hz, 2H, Ar), 7.92 (d, *J =* 8.2 Hz, 1H, NHCHCH₂), 8.10 (d, *J =* 8.9 Hz, 2H, Ar), 11.70 (br s, 1H, ArNH).

**Methyl (S)-2-((((9H-fluoren-9-yl)methoxy)carbonyl)amino)-3-(2-((3-nitrophenyl)thio)-1H-indol-3-yl)propanoate (3b).** Following the general procedure for sulfenylation, Fmoc tryptophan methyl ester **2** (430 mg, 0.97 mmol), commercially available 3-nitrophenyldisulfide (200 mg, 0.65 mmol) and I₂ (168 mg, 0.66 mmol) in acetonitrile (2.6 mL) reacted for 4 hours. After workup, the crude product was subjected to column chromatography (DCM/ethyl acetate, 40:1) to yield 310 mg (80%) of **3b** as a yellow solid. Mp 121-123 °C. MS (ES, positive mode): m/z 594 (M+H).^{+ 1}H NMR (400 MHz, DMSO-*d₆*) *δ*: 3.20 (dd, *J* = 14.2, 8.3 Hz, 1H, NHCHCH₂), 3.28 (m, 1H, NHCHCH₂), 3.48 (s, 3H, COOCH₃), 4.09 - 4.19 (m, 3H, COOCH₂CH), 4.24 (q, *J* = 7.8 Hz, 1H, NHCHCH₂), 7.07 (t, *J* = 7.5 Hz, 1H, Ar), 7.21 (t, *J* = 7.3 Hz, 1H, Ar), 7.25 - 7.32 (m, 2H, Ar), 7.36 (d, *J* = 8.2 Hz, 1H, Ar), 7.38 - 7.44 (m, 3H, Ar), 7.48 (dt, *J* = 8.0, 1.3 Hz, 1H, Ar), 7.56 (t, *J* = 8.0 Hz, 1H, Ar), 7.63 (t, *J* = 6.9 Hz, 2H, Ar), 7.70 (d, *J* = 8.0 Hz, 1H, Ar), 7.79 (t, *J* = 2.1 Hz, 1H, Ar), 7.88 (d, *J* = 7.5 Hz, 2H, Ar), 7.91 (d, *J* = 8.2 Hz, 1H, Ar), 7.99 (dd, *J* = 8.1, 1.5 Hz, 1H, Ar), 11.67 (br s, 1H, ArNH).

**Methyl (S)-2-((((9H-fluoren-9-yl)methoxy)carbonyl)amino)-3-(2-((4-acetylphenyl)thio)-1H-indol-3-yl)propanoate (3c).** Following the general procedure for sulfenylation, Fmoc tryptophan methyl ester **2** (486 mg, 1.10 mmol), 4-acetylphenyldisulfide (371 mg, 1.23 mmol) and l₂ (187 mg, 0.74 mmol) in acetonitrile (4.4 mL) reacted for 2.5 hours. After workup, the crude product was subjected to column chromatography (hexane/ethyl acetate, 2:1) to yield 382 mg (59%) of **3c** as a pale yellow solid. Mp 100-102 °C. MS (ES, positive mode): m/z 591 (M+H).^{+ 1}H NMR (400 MHz, DMSO-*d₆*) *δ*: 2.47 (s, 3H, COCH₃), 3.18 (dd, *J* = 14.2, 8.2 Hz, 1H, NHCHCH₂), 3.28 (dd, *J* = 14.3, 7.0 Hz, 1H, NHCHCH₂), 3.48 (s, 3H, COOCH₃), 4.10 - 4.20 (m, 3H, COOCH₂CH), 4.25 (q, *J* = 7.9 Hz, 1H, NHCHCH₂), 7.05 (t, *J* = 7.5 Hz, 1H, Ar), 7.09 (d, *J* = 8.5 Hz, 2H, Ar), 7.19 (t, *J* = 7.7 Hz, 1H, Ar), 7.25 - 7.36 (m, 3H, Ar), 7.39 (m, 2H, Ar), 7.63 (t, *J* = 8.5 Hz, 2H, Ar), 7.68 (d, *J* = 8.0 Hz, 1H, Ar), 7.82 (d, *J* = 8.3 Hz, 2H, Ar), 7.88 (d, *J* = 7.6 Hz, 2H, Ar), 7.96 (d, *J* = 8.1 Hz, 1H, NHCHCH₂), 11.64 (br s, 1H, ArNH).

**Methyl (S)-2-((((9H-fluoren-9-yl)methoxy)carbonyl)amino)-3-(2-((4-(trifluoromethyl)phenyl)thio)-1H-indol-3-yl)propanoate (3d).** Following the general procedure for sulfenylation, Fmoc tryptophan methyl ester **2** (282 mg, 0.64 mmol), 4-trifluoromethylphenyldisulfide (as described in Zheng, Y.; Qing, F.-L.; Huang, Y.; Xu, X.-H. Tunable and Practical Synthesis of Thiosulfonates and Disulfides from Sulfonyl Chlorides in the Presence of Tetrabutylammonium Iodide. Adv. Synth. Catal. 2016, 358 (21), 3477-3481) (170 mg, 0.48 mmol) and l₂ (73 mg, 0.29 mmol) in acetonitrile (1.9 mL) reacted for 3 hours. After workup, the crude product was purified by CCTLC in the Chromatotron (hexane/ethyl acetate, 3:1) to yield 245 mg (83%) of 3d as a pale yellow solid. Mp 85-87 °C. MS (ES, positive mode): m/z 617 (M+H).^{+ 1}H NMR (400 MHz, DMSO-*d₆*) *δ*: 3.19 (dd, *J* = 14.2, 8.2 Hz, 1H, NHCHCH₂), 3.28 (m, 1H, NHCHCH₂), 3.49 (s, 3H, COOCH₃), 4.10 - 4.21 (m, 3H, COOCH₂CH), 4.27 (q, *J* = 7.7 Hz, 1H, NHCHCH₂), 7.06 (ddd, *J* = 8.0, 6.9, 1.0 Hz, 1H, Ar), 7.15 - 7.22 (m, 3H, Ar), 7.24 - 7.44 (m, 5H, Ar), 7.60 (d, *J* = 8.3 Hz, 2H, Ar), 7.62 - 7.71 (m, 3H, Ar), 7.88 (d, *J* = 7.7 Hz, 2H, Ar), 7.93 (d, *J* = 8.1 Hz, 1H, NHCHCH₂), 11.62 (br s, 1H, ArNH).

**Methyl (S)-2-((((9H-fluoren-9-yl)methoxy)carbonyl)amino)-3-(2-((4-cyanophenyl)thio)-1H-indol-3-yl)propanoate (3e).** Following the general procedure for sulfenylation, Fmoc tryptophan methyl ester **2** (431 mg, 0.98 mmol), commercially available 4-cyanophenyldisulfide (175 mg, 0.65 mmol) and l₂ (99 mg, 0.39 mmol) in acetonitrile (2.6 mL) reacted for 4 hours. After workup, the crude product was subjected to column chromatography (DCM/ethyl acetate, 70:1) to yield 206 mg (55%) of 3e as a white solid. Mp 102-104 °C. MS (ES, positive mode): m/z 574 (M+H).^{+ 1}H NMR (400 MHz, DMSO-*d₆*) *δ*: 3.18 (dd, J= 14.1, 8.2 Hz, 1H, NHCHCH₂), 3.28 (dd, *J* = 14.1, 6.8 Hz, 1H, NHCHCH₂), 3.48 (s, 3H, COOCH₃), 4.10 - 4.21 (m, 3H, COOCH₂CH), 4.26 (q, *J* = 7.9 Hz, 1H, NHCHCH₂), 7.07 (t, *J* = 7.5 Hz, 1H, Ar), 7.12 (d, *J* = 8.6 Hz, 2H, Ar), 7.20 (ddd, *J* = 8.2, 7.0, 1.1 Hz, 1H, Ar), 7.29 (m, 2H, Ar), 7.35 (d, *J* = 8.2 Hz, 1H, Ar), 7.41 (td, *J* = 7.5, 3.4 Hz, 2H, Ar), 7.64 (t, *J* = 8.8 Hz, 2H, Ar), 7.66 - 7.73 (m, 3H, Ar), 7.88 (d, *J* = 7.6 Hz, 2H, Ar), 7.92 (d, *J* = 8.1 Hz, 1H, NHCHCH₂), 11.65 (br s, 1H, ArNH).

**Methyl (S)-2-(**((**(9H-fluoren-9-yl)methoxy)carbonyl)amino)-3-(2-((4-fluorophenyl)thio)-1H-indol-3-yl)propanoate (3f).** Following the general procedure for sulfenylation, Fmoc tryptophan methyl ester **2** (538 mg, 1.22 mmol), commercially available 4-fluorophenyldisulfide (207 mg, 0.81 mmol) and l₂ (124 mg, 0.49 mmol) in acetonitrile (3.2 mL) reacted for 6 hours. After workup, the crude product was subjected to column chromatography (DCM/ethyl acetate, 70:1) to yield 311 mg (72%) of **3f** as a white solid. Mp 76-78 °C. MS (ES, positive mode): m/z 567 (M+H).^{+ 1}H NMR (400 MHz, DMSO-*d₆*) *δ*: 3.18 (dd, *J =* 14.1, 8.1 Hz, 1H, NHCHCH₂), 3.29 (m, 1H, NHCHCH₂), 3.49 (s, 3H, COOCH₃), 4.12 - 4.22 (m, 3H, COOCH₂CH), 4.25 (q, *J =* 7.7 Hz, 1H, NHCHCH₂), 7.03 (t, *J =* 7.5 Hz, 1H, Ar), 7.13 (d, *J* = 6.9 Hz, 4H, Ar), 7.16 (m, 1H, Ar), 7.25 - 7.35 (m, 3H, Ar), 7.41 (m, 2H, Ar), 7.65 (m, 3H, Ar), 7.88 (d, *J* = 7.8 Hz, 2H, Ar), 7.92 (d, *J* = 8.1 Hz, 1H, NHCHCH₂), 11.51 (br s, 1H, ArNH).

**General procedure for selective Fmoc deprotection (compounds 4a-f).** The appropriate Fmoc protected Trp derivative (3a-f) (1 mmol) was dissolved in DCM (10 mL) and then piperidine (10 mmol) was added dropwise. The reaction was stirred at rt for 2-3 hours. Volatiles were removed and the residue was purified as indicated for each compound.

**Methyl (*S*)-2-amino-3-(2-((4-nitrophenyl)thio)-1H-indol-3-yl)propanoate (4a).** Following the general procedure for selective Fmoc deprotection, 3a (460 mg, 0.77 mmol) and piperidine (765 µL, 7.75 mmol) in DCM (8.0 mL) reacted for 2 hours. After workup, the residue was subjected to column chromatography (DCM:hexane:methanol, 10:10:0.6) to yield 244 mg (85%) of **4a** as a yellow solid. Mp 73-75 °C. MS (ES, positive mode): m/z 372 (M+H).^{+ 1}H NMR (400 MHz, DMSO-*d₆*) δ: 2.98 (dd, *J* = 13.9, 7.0 Hz, 1H, NH₂CHCH₂), 3.12 (dd, *J* = 13.8, 6.9 Hz, 1H, NH₂CHCH₂), 3.47 (s, 3H, COOCH₃), 3.57 (t, *J* = 6.9 Hz, 1H, NH₂CHCH₂), 7.09 (t, *J* = 5.0 Hz1H, Ar), 7.17 - 7.23 (m, 3H, Ar), 7.36 (d, *J* = 8.3 Hz, 1H, Ar), 7.63 (d, *J* = 8.0 Hz, 1H, Ar), 8.13 (d, *J =* 9.0 Hz, 2H, Ar), 11.65 (br s, 1H, ArNH).

**Methyl (*S*)-2-amino-3-(2-((3-nitrophenyl)thio)-1H-indol-3-yl)propanoate (4b).** Following the general procedure for selective Fmoc deprotection, **3b** (277 mg, 0.47 mmol) and piperidine (461 µL, 4.67 mmol) in DCM (4.7 mL) reacted for 1.5 hours. After workup, the residue was purified by CCTLC in the Chromatotron (DCM:hexane:methanol, 10:10:0.7) to yield 133 mg (89%) of **4b** as a yellow solid. Mp 80-82 °C. MS (ES, positive mode): m/z 372 (M+H).^{+ 1}H NMR (400 MHz, DMSO-*d₆*) *δ*: 3.01 (dd, *J* = 13.8, 7.0 Hz, 1H, NH₂CHCH₂), 3.14 (dd, *J* = 13.8, 7.0 Hz, 1H, NH₂CHCH₂), 3.44 (s, 3H, COOCH₃), 3.56 (t, *J =* 7.0 Hz, 1H, NH₂CHCH₂), 7.08 (t, 1H, Ar), 7.20 (ddd, *J* = 8.2, 6.9, 1.1 Hz, 1H, Ar), 7.35 (d, *J* = 8.2 Hz, 1H, Ar), 7.49 (dt, *J* = 8.0, 1.4 Hz, 1H, Ar), 7.58 (t, *J* = 8.1 Hz, 1H, Ar), 7.62 (d, *J* = 8.2 Hz, 1H, Ar), 7.81 (t, *J* = 2.0 Hz, 1H, Ar), 8.00 (dd, *J* = 7.8, 2.1 Hz, 1H, Ar), 11.61 (br s, 1H, ArNH).

**Methyl (*S*)-3-(2-((4-acetylphenyl)thio)-1H-indol-3-yl)-2-aminopropanoate (4c).** Following the general procedure for selective Fmoc deprotection, **3c** (451 mg, 0.76 mmol) and piperidine (787 µL, 7.63 mmol) in DCM (7.6 mL) reacted for 3 hours. After workup, the residue was purified by CCTLC in the Chromatotron (DCM:methanol, 30:1) to yield 203 mg (83%) of **4c** as a pale yellow solid. Mp 79-81 °C. MS (ES, positive mode): m/z 369 (M+H).^{+ 1}H NMR (400 MHz, DMSO-*d₆*) *δ*: 2.99 (dd, *J* = 12.8, 7.0 Hz, 1H, NH₂CHCH₂), 3.12 (dd, *J* = 13.8, 6.9 Hz, 1H, NH₂CHCH₂), 3.46 (s, 3H, COOCH₃), 3.57 (t, *J =* 6.9 Hz, 1H, NH₂CHCH₂), 7.01 - 7.13 (m, 3H, Ar), 7.19 (ddd, *J* = 8.2, 7.0, 1.2 Hz, 1H, Ar), 7.34 (d, 1H, Ar), 7.61 (d, *J* = 8.0 Hz, 1H, Ar), 7.84 (dd, *J* = 9.3, 2.7 Hz, 2H, Ar).

**Methyl (*S*)-2-amino-3-(2-((4-(trifluoromethyl)phenyl)thio)-1H-indol-3-yl)propanoate (4d).** Following the general procedure for selective Fmoc deprotection, 3d (237 mg, 0.38 mmol) and piperidine (379 µL, 3.84 mmol) in DCM (3.8 mL) reacted for 2 hours. After workup, the residue was purified by CCTLC in the Chromatotron (DCM:hexane:methanol, 10:10:0.5) to yield 103 mg (68%) of **4d** as an amorphous pale yellow solid. MS (ES, positive mode): m/z 395 (M+H).^{+ 1}H NMR (400 MHz, DMSO-*d₆*) *δ*: 2.99 (dd, *J* = 13.8, 7.0 Hz, 1H, NH₂CHCH₂), 3.13 (dd, *J* = 13.8, 6.9 Hz, 1H, NH₂CHCH₂), 3.46 (s, 3H, COOCH₃), 3.57 (t, *J* = 6.9 Hz, 1H, NH₂CHCH₂), 7.07 (ddd, *J* = 8.1, 6.9, 1.1 Hz, 1H, Ar), 7.16 - 7.22 (m, 3H, Ar), 7.34 (d, *J* = 8.2 Hz, 1H, Ar), 7.59 - 7.66 (m, 3H, Ar), 11.56 (br s, 1H, ArNH).

**Methyl (*S*)-2-amino-3-(2-((4-cyanophenyl)thio)-1H-indol-3-yl)propanoate (4e).** Following the general procedure for selective Fmoc deprotection, **3e** (196 mg, 0.34 mmol) and piperidine (337 µL, 3.42 mmol) in DCM (3.4 mL) reacted for 3 hours. After workup, the residue was purified by CCTLC in the Chromatotron (DCM:hexane:methanol, 10:10:1.4) to yield 98 mg (82%) of **4e** as a pale yellow solid. Mp 72-74 °C. MS (ES, positive mode): m/z 352 (M+H).^{+ 1}H NMR (500 MHz, DMSO-*d₆*) *δ*: 2.97 (dd, *J* = 13.9, 7.0 Hz, 1H, NH₂CHCH₂), 3.11 (dd, *J* = 13.9, 7.0 Hz, 1H, NH₂CHCH₂), 3.46 (s, 3H, COOCH₃), 3.56 (t, *J* = 6.9 Hz, 1H, NH₂CHCH₂), 7.07 (ddd, *J* = 8.1, 7.0, 1.1 Hz, 1H, Ar), 7.13 (m, 2H, Ar), 7.20 (ddd, *J* = 8.2, 7.0, 1.2 Hz, 1H, Ar), 7.34 (dd, *J* = 8.2, 1.0 Hz, 1H, Ar), 7.62 (dd, *J* = 8.1, 1.1 Hz, 1H, Ar), 7.72 (m, 2H, Ar), 11.60 (br s, 1H, ArNH).

**Methyl (*S*)-2-amino-3-(2-((4-fluorophenyl)thio)-1H-indol-3-yl)propanoate (4f).** Following the general procedure for selective Fmoc deprotection, 3f (303 mg, 0.53 mmol) and piperidine (528 µL, 5.35 mmol) in DCM (5.3 mL) reacted for 2 hours. After workup, the residue was purified by CCTLC in the Chromatotron (DCM:hexane:methanol, 10:10:0.6) to yield 140 mg (76%) of **4f** as an amorphous white solid. MS (ES, positive mode): m/z 345 (M+H).^{+ 1}H NMR (400 MHz, DMSO-*d₆*) *δ*: 3.00 (dd, *J* = 13.8, 7.0 Hz, 1H, NH₂CHCH₂), 3.13 (dd, *J* = 13.8, 6.9 Hz, 1H, NH₂CHCH₂), 3.48 (s, 3H, COOCH₃), 3.57 (t, *J* = 6.9 Hz, 1H, NH₂CHCH₂), 7.03 (ddd, *J* = 8.1, 7.0, 1.1 Hz, 1H, Ar), 7.07 - 7.20 (m, 5H, Ar), 7.31 (dd, *J* = 8.1, 1.0 Hz, 1H, Ar), 7.57 (d, *J* = 7.9 Hz, 1H, Ar), 11.45 (br s, 1H, ArNH).

### General procedure for the coupling to the central scaffold (trimers 6a-6f).

To a solution containing the tripodal acid (5) (Weissenborn, M. J.; Castangia, R.; Wehner, J. W.; Šardzík, R.; Lindhorst, T. K.; Flitsch, S. L. Oxo-Ester Mediated Native Chemical Ligation on Microarrays: An Efficient and Chemoselective Coupling Methodology. Chem. Commun. 2012, 48 (37), 4444-4446) (1.0 mmol), HATU (1.3 equiv for each carboxylic acid group), the corresponding amine **4a-f** (1.1-1.2 equiv. each carboxylic acid group) in DMF (10 mL), DIPEA (2.3 equiv. each carboxylic acid group) was added. The reaction mixture was stirred at 30 °C for 24-48 h. Then it was quenched with a saturated solution of NH₄Cl (5 mL) and volatiles were removed. The residue was dissolved in ethyl acetate (20 mL) and washed with water (3 x 20 mL). The organic phase was dried over anhydrous Na₂SO₄, filtered and evaporated to dryness. The residue was purified by CCTLC on Chromatotron to give the corresponding OMe ester compound.

**Trimer 6a.** Following the general coupling procedure triacid **5** (105 mg, 0.20 mmol), **4a** (244 mg, 0.66 mmol), HATU (303 mg, 0.80 mmol) and DIPEA (242 µL, 1.39 mmol) in anhydrous DMF (2.0 mL) reacted for 24 hours. After workup, the residue was purified by CCTLC in the Chromatotron (DCM:methanol, 25:1) to yield 282 mg (89%) of **6a** as a yellow solid. Mp (decomp at 135 °C). ¹H NMR (400 MHz, CDCl₃) *δ*: 1.51 (m, 3H, CH₂CH₂CO), 1.72 (m, 3H, CH₂CH₂CO), 1.95 (m, 6H, CH₂CH₂CO), 2.83 (s, 2H, NHCOCH₂NH), 3.19 (dd, *J* = 14.3, 7.4 Hz, 3H, NHCHCH₂), 3.38 (dd, *J* = 14.4, 5.3 Hz, 3H, NHCHCH₂), 3.64 (s, 9H, COOCH₃), 4.15 (t, *J* = 7.1 Hz, 1H, COOCH₂CH), 4.30 (m, 2H, COOCH₂CH), 4.87 (m, 3H, NHCHCH₂), 5.39 (br s, 1H, NHCOCH₂NH), 6.61 (br s, 3H, NHCHCH₂), 6.97 (d, *J* = 8.5 Hz, 6H, Ar), 7.11 (t, *J* = 7.4 Hz, 3H, Ar), 7.19 (t, J = 7.5 Hz, 3H, Ar), 7.22 - 7.25 (m, 6H, Ar, NHCOCH₂NH), 7.36 (q, *J* = 6.9 Hz, 2H, Ar), 7.55 (m, 5H, Ar), 7.72 (dd, *J* = 7.6, 3.2 Hz, 2H, Ar), 7.93 (d, *J* = 8.2 Hz, 6H, Ar), 9.09 (br s, 3H, ArNH). ¹³C NMR (101 MHz, CDCl₃) δ: 26.5 (NHCHCH₂), 29.3 (CH₂CH₂CO), 29.9 (CH₂CH₂CO), 37.8 (NHCOCH₂NH), 46.0 (COOCH₂CH), 51.6 (COOCH₃), 51.7 (NHCHCH₂), 56.7 (C(NHCOCH₂NH)), 66.2 (COOCH₂CH), 110.7, 117.9, 118.3, 119.0, 119.5, 119.6, 123.2, 123.3, 124.0, 124.1, 124.7, 126.1, 126.5, 126.8, 136.3, 140.2, 142.6, 142.8, 144.6, 145.4 (Ar), 155.9 (COOCH₂CH), 171.1 (COOCH₃), 172.1 (CH₂CH₂CO). HRMS (ESI⁺) m/z: calc for C₈₁H₇₅N₁₁O₁₈S₃ 1585.4454; found 1586.4521 (M+H)⁺.

**Trimer 6b.** Following the general coupling procedure triacid **5** (55 mg, 0.11 mmol), **4b** (129 mg, 0.35 mmol), HATU (160 mg, 0.42 mmol) and DIPEA (128 µL, 0.74 mmol) in anhydrous DMF (1.0 mL) reacted for 24 hours. After workup, the residue was purified by CCTLC in the Chromatotron (DCM:methanol, 44:1) to yield 87 mg (52%) of **6b** as a yellow solid. Mp (decomp at 124 °C). ¹H NMR (400 MHz, CDCl₃) *δ*: 1.50 (m, 3H, CH₂CH₂CO), 1.69 (s, 3H, CH₂CH₂CO), 1.95 (s, 6H, CH₂CH₂CO), 3.22 (dd, J = 14.3, 8.0 Hz, 3H, NHCHCH₂), 3.42 (dd, J = 14.3, 5.4 Hz, 3H, NHCHCH₂), 3.55 (m, 2H, NHCOCH₂NH), 3.65 (s, 9H, COOCH₃), 4.15 (t, J = 7.2 Hz, 1H, COOCH₂CH), 4.29 (m, 2H, COOCH₂CH), 4.88 (d, J = 6.6 Hz, 3H, NHCHCH₂), 5.47 (br s, 1H, NHCOCH₂NH), 6.71 (br s, 3H, NHCHCH₂), 7.09 (t, J = 7.4 Hz, 3H, Ar), 7.15 - 7.25 (m, 14H, Ar), 7.36 (t, 2H, Ar), 7.52 - 7.61 (m, 5H, Ar), 7.72 (d, J = 7.5 Hz, 2H, Ar), 7.84 - 7.90 (m, 6H), 9.06 (br s, 3H, ArNH). HRMS (ESI⁺) m/z: calc for C₈₁H₇₅N₁₁O₁₈S₃ 1585.4454; found 1586.4548 (M+H)+.

**Trimer 6c.** Following the general coupling procedure triacid **5** (56 mg, 0.11 mmol), **4c** (150 mg, 0.41 mmol), HATU (161 mg, 0.42 mmol) and DIPEA (129 µL, 0.74 mmol) in anhydrous DMF (1.1 mL) reacted for 24 hours. After workup, the residue was purified by CCTLC in the Chromatotron (DCM:methanol, 28:1) to yield 95 mg (57%) of **6c** as a pale yellow solid. Mp (decomp at 137 °C). ¹H NMR (400 MHz, CDCl₃) *δ*: 1.54 (m, 3H, CH₂CH₂CO), 1.72 (m, 3H, CH₂CH₂CO), 1.90 (m, 6H, CH₂CH₂CO), 2.45 (s, 9H, COCH₃), 3.21 (dd, *J* = 14.2, 7.6 Hz, 3H, NHCHCH₂), 3.38 (dd, *J* = 14.3, 5.4 Hz, 3H, NHCHCH₂), 3.51 (m, 2H, NHCOCH₂NH), 3.63 (s, 9H, COOCH₃), 4.14 (t, *J* = 7.0 Hz, 1H, COOCH₂CH), 4.30 (d, *J* = 7.0 Hz, 2H, COOCH₂CH), 4.87 (td, *J* = 7.8, 5.5 Hz, 3H, NHCHCH₂), 5.41 (br s, 1H, NHCOCH₂NH), 6.43 (d, *J* = 8.1 Hz, 3H, NHCHCH₂), 6.97 (d, *J* = 8.4 Hz, 6H, Ar), 7.10 (td, *J* = 7.4, 6.8, 1.2 Hz, 3H, Ar), 7.18 (d, 3H, *J* = 7.6 Hz, Ar), 7.21 - 7.25 (m, 4H, Ar), 7.36 (td, *J* = 7.5, 2.3 Hz, 3H, Ar), 7.51 - 7.61 (m, 5H, Ar, NHCOCH₂NH), 7.69 (d, *J* = 8.4 Hz, 6H), 7.73 (d, *J* = 7.7 Hz, 3H), 9.01 br (s, 3H, ArNH). ¹³C NMR (101 MHz, CDCl₃) δ: 25.4 (COCH₃), 26.4 (NHCHCH₂), 29.4 (CH₂CH₂CO), 29.8 (CH₂CH₂CO), 43.5 (NHCOCH₂NH), 46.1 (COOCH₂CH), 51.5 (COOCH₃), 51.7 (NHCHCH₂), 56.5 (C(NHCOCH₂NH)), 66.1 (COOCH₂CH), 117.4, 118.2, 110.5, 118.9, 119.3, 120.5, 123.0, 124.1, 124.6, 126.1, 126.7, 128.0, 133.5, 136.2, 140.2, 142.6, 142.8, 142.9 (Ar), 155.7 (COOCH₂CH), 167.4 (NHCOCH₂NH), 171.3 (COOCH₃), 171.7 (CH₂CH₂CO), 196.2 (COCH₃). HRMS (ESI⁺) m/z: calc for C₈₇H₈₄N₈O₁₅S₃ 1576.5218; found 1577.5305 (M+H)⁺.

**Trimer 6d.** Following the general coupling procedure triacid **5** (34 mg, 0.06 mmol), **4d** (92 mg, 0.23 mmol), HATU (99 mg, 0.26 mmol) and DIPEA (80 µL, 0.46 mmol) in anhydrous DMF (0.7 mL) reacted for 24 hours. After workup, the residue was purified by CCTLC in the Chromatotron (DCM:methanol, 25:1) to yield 73 mg (68%) of 6d as a white solid. Mp (decomp at 126 °C). ¹H NMR (400 MHz, CDCl₃) *δ*: 1.54 (m, 3H, CH₂CH₂CO), 1.75 (m, 3H, CH₂CH₂CO), 1.93 (m, 6H, CH₂CH₂CO), 2.80 (s, 2H, NHCOCH₂NH), 3.21 (dd, *J* = 14.3, 7.6 Hz, 3H, NHCHCH₂), 3.39 (dd, *J* = 14.4, 5.5 Hz, 3H, NHCHCH₂), 3.64 (s, 9H, COOCH₃), 4.18 (t, *J* = 7.0 Hz, 1H, COOCH₂CH), 4.32 (m, 2H, COOCH₂CH), 4.90 (td, *J* = 7.8, 5.4 Hz, 3H, NHCHCH₂), 5.29 (br s, 1H, NHCOCH₂NH), 6.43 (d, J= 8.2 Hz, 3H, NHCHCH₂), 7.01 (d, J= 8.2 Hz, 6H, Ar), 7.10 (ddd, *J* = 8.0, 6.2, 1.8 Hz, 2H, Ar), 7.14 - 7.22 (m, 6H, Ar), 7.23 - 7.25 (m, 3H, Ar, NHCOCH₂NH), 7.37 (m, 8H, Ar), 7.52 - 7.62 (m, 5H, Ar), 7.74 (dd, *J* = 7.5, 2.2 Hz, 2H, Ar), 8.93 (br s, 3H, ArNH). ¹³C NMR (101 MHz, CDCl₃) δ: 27.6 (NHCHCH₂), 30.5 (CH₂CH₂CO), 31.0 (CH₂CH₂CO), 38.7 (NHCOCH₂NH), 47.2 (COOCH₂CH), 52.6 (COOCH₃), 52.7 (NHCHCH₂), 57.6 (C(NHCOCH₂NH)), 67.2 (COOCH₂CH), 111.6, 118.5, 119.3, 120.1, 120.4, 121.8, 124.0 (q, *J* = 271.9 Hz), 124.1, 125.2, 126.0 (q, *J* = 3.7 Hz), 126.2, 127.2, 127.7, 127.8, 128.0 (q, *J* = 31.5 Hz), 137.3, 141.4, 141.8, 143.9, 144.0 (Ar, CF₃), 156.8 (COOCH₂CH), 168.5 (NHCOCH₂NH), 172.4 (COOCH₃), 172.9 (CH₂CH₂CO).

**Trimer 6e.** Following the general coupling procedure triacid **5** (39 mg, 0.07 mmol), **4e** (86 mg, 0.25 mmol), HATU (112 mg, 0.30 mmol) and DIPEA (90 µL, 0.52 mmol) in anhydrous DMF (0.8 mL) reacted for 24 hours. After workup, the residue was purified by CCTLC in the Chromatotron (DCM:methanol, 22:1) to yield 74 mg (65%) of **6e** as a pale yellow solid. Mp (decomp at 115 °C). ¹H NMR (400 MHz, CDCl₃) *δ*: 1.81 (m, 6H, CH₂CH₂CO), 1.93 (m, 6H, CH₂CH₂CO), 2.87 (s, 2H, NHCOCH₂NH), 3.18 (dd, J= 14.3, 7.8 Hz, 3H, NHCHCH₂), 3.37 (dd, *J* = 14.3, 5.4 Hz, 3H, NHCHCH₂), 3.64 (s, 9H, COOCH₃), 4.16 (t, *J* = 7.0 Hz, 1H, COOCH₂CH), 4.30 (m, 2H, COOCH₂CH), 4.86 (q, *J* = 7.3 Hz, 3H, NHCHCH₂), 5.49 (br s, 1H, NHCOCH₂NH), 6.47 (d, *J* = 8.2 Hz, 3H, NHCHCH₂), 6.96 (d, *J* = 8.2 Hz, 6H, Ar), 7.10 (t, *J* = 7.4 Hz, 3H, Ar), 7.19 (t, *J* = 7.4 Hz, 3H), 7.36 (m, 8H, Ar), 7.52 - 7.60 (m, 5H, Ar), 7.23 - 7.25 (m, 6H, Ar, NHCOCH₂NH), 7.74 (d, *J* = 7.6 Hz, 2H, Ar), 9.18 (br s, 3H, ArNH). HRMS (ESI⁺) m/z: calc for C₈₄H₇₅N₁₁O₁₂S₃ 1525.4759; found 1526.4844 (M+H)⁺.

**Trimer 6f.** Following the general coupling procedure triacid 5 (63 mg, 0.12 mmol), **4f** (135 mg, 0.39 mmol), HATU (181 mg, 0.48 mmol) and DIPEA (145 µL, 0.83 mmol) in anhydrous DMF (1.2 mL) reacted for 24 hours. After workup, the residue was purified by CCTLC in the Chromatotron (DCM:methanol, 28:1) to yield 133 mg (74%) of 6f as a pale yellow solid. Mp (decomp at 107 °C). ¹H NMR (400 MHz, DMSO-*d₆*) *δ*: 1.75 (m, 6H, CH₂CH₂CO), 1.99 (m, 6H, CH₂CH₂CO), 3.14 (dd, *J* = 13.9, 6.7 Hz, 3H, NHCHCH₂), 3.25 (dd, *J =* 14.0, 8.0 Hz, 3H, NHCHCH₂), 3.41 (s, 9H, COOCH₃), 3.58 (d, J= 5.3 Hz, 2H, NHCOCH₂NH), 4.18 (m, 1H, COOCH₂CH), 4.26 (d, *J* = 7.0 Hz, 2H, COOCH₂CH), 4.46 (q, *J =* 7.4 Hz, 3H, NHCHCH₂), 7.03 (t, *J =* 7.3 Hz, 3H, Ar), 7.08 - 7.22 (m, 16H), 7.29 (dd, *J* = 7.9, 2.3 Hz, 5H), 7.38 (q, *J* = 6.8, 6.0 Hz, 3H), 7.56 (d, *J* = 8.0 Hz, 3H), 7.72 - 7.66 (m, 2H), 7.87 (d, *J* = 7.6 Hz, 2H), 8.29 (d, *J* = 7.5 Hz, 3H), 11.48 (s, 3H). HRMS (ESI⁺) m/z: calc for C₈₁H₇₅F₃N₈O₁₂S₃ 1504.4619; found 1505.4683 (M+H)⁺.

### General procedure for methyl ester deprotection (trimers 7a-f).

To a solution containing the corresponding methyl ester derivative **(6a-f)** (1.0 mmol) in THF (20 mL) at 0 °C (ice-bath), a solution of LiOH·H₂O (2 equiv. for each methyl ester group) in water (4 mL) was added and the mixture was stirred at room temperature overnight. Then, 1 N hydrochloric acid aqueous solution was added to reach pH=2, and volatiles were evaporated to dryness. The residue was dissolved in ethyl acetate (15 mL) and washed with H₂O (3 x 10 mL). The organic phase was dried over anhydrous Na₂SO₄, filtered, and evaporated to dryness. The residue was purified as indicated for each compound.

**Trimer 7a.** Following the general procedure for methyl ester deprotection, a solution of **6a** (46 mg, 0.03 mmol) in THF (0.6 mL) and LiOH·H₂O (8 mg, 0.17 mmol) in water (0.2 mL) were stirred at room temperature overnight. After workup, the residue was precipitated with cool diethyl ether to afford 38 mg (94% yield) of **7a** as a yellow solid. Mp (decomp at 195 °C). ¹H NMR (400 MHz, DMSO-*d₆*) *δ*: 1.70 (m, 6H, CH₂CH₂CO), 1.97 (m, 6H, CH₂CH₂CO), 3.12 (dd, *J* = 14.0, 7.3 Hz, 3H, NHCHCH₂), 3.25 (dd, *J* = 14.1, 6.9 Hz, 3H, NHCHCH₂), 3.45 (s, 2H, NHCOCH₂NH₂), 4.47 (q, *J* = 7.3 Hz, 3H, NHCHCH₂), 7.06 (t, *J* = 7.5 Hz, 3H, Ar), 7.12 - 7.23 (m, 9H, Ar), 7.33 (d, *J* = 8.2 Hz, 3H, Ar), 7.66 (br s, 1H, NHCOCH₂NH₂), 7.72 (d, *J* = 8.0 Hz, 3H, Ar), 8.09 (d, *J* = 9.0 Hz, 6H, Ar), 8.13 (d, *J* = 7.9 Hz, 3H, NHCHCH₂), 11.64 (br s, 3H, ArNH). ¹³C NMR (101 MHz, DMSO-*d₆*) *δ*: 27.9 (NHCHCH₂), 29.8 (CH₂CH₂CO), 30.6 (CH₂CH₂CO), 40.9 (NHCOCH₂NH₂), 53.8 (NHCHCH₂), 57.9 (C(NHCOCH₂NH₂), 112.1, 119.8, 119.9, 120.1, 120.2, 123.8, 124.8, 126.3, 127.9, 138.0, 145.5, 148.1 (Ar), 165.6 (NHCOCH₂NH₂), 172.2 (CH₂CH₂CO), 173.6 (COOH). HRMS (ESI⁺) m/z: calc for C₆₃H₅₉N₁₁O₁₆S₃ 1321.3303; found 1322.3378 (M+H)⁺.

**Trimer 7b.** Following the general procedure for methyl ester deprotection, a solution of **6b** (75 mg, 0.05 mmol) in THF (0.9 mL) and LiOH·H₂O (12 mg, 0.28 mmol) in water (0.2 mL) were stirred at room temperature overnight. After workup, the residue was precipitated with cool diethyl ether to afford 58 mg (93%) of **7b** as a yellow solid. Mp (decomp at 182 °C). ¹H NMR (400 MHz, DMSO-*d₆*) *δ*: 1.65 (m, 6H, CH₂CH₂CO), 1.96 (m, 6H, CH₂CH₂CO), 3.13 (dd, *J* = 13.9, 7.3 Hz, 3H, NHCHCH₂), 3.28 (dd, *J* = 14.0, 6.8 Hz, 3H, NHCHCH₂), 3.43 (s, 2H, NHCOCH₂NH₂), 4.46 (q, *J* = 7.4 Hz, 3H, NHCHCH₂), 7.06 (t, *J =* 7.5 Hz, 3H, Ar), 7.16 (ddd, *J* = 8.1, 6.9, 1.2 Hz, 3H, Ar), 7.32 (d, *J* = 8.1 Hz, 3H, Ar), 7.45 (dt, *J* = 7.9, 1.4 Hz, 3H, Ar), 7.53 (t, *J* = 8.0 Hz, 3H, Ar), 7.62 (s, 1H, NHCOCH₂NH₂), 7.72 (d, *J =* 8.0 Hz, 3H, Ar), 7.80 (t, *J* = 2.0 Hz, 3H, Ar), 7.97 (ddd, *J* = 8.1, 2.4, 1.1 Hz, 3H, Ar), 8.05 (d, *J* = *8.1* Hz, 3H, NHCHCH₂), 11.58 (br s, 3H, ArNH). ¹³C NMR (101 MHz, DMSO-*d₆*) *δ*: 27.4 (NHCHCH₂), 29.3 (CH₂CH₂CO), 30.0 (CH₂CH₂CO), 53.4 (NHCHCH₂), 57.4 (C(NHCOCH₂NH₂)), 111.4, 119.0, 119.3, 119.6, 120.2, 120.5, 120.6, 123.1, 127.3, 130.5, 132.3, 137.4, 140.1, 148.3 (Ar), 165.1 (NHCOCH₂NH₂), 171.7 (CH₂CH₂CO), 173.1 (COOH). HRMS (ESI⁺) m/z: calc for C₆₃H₅₉N₁₁O₁₆S₃ 1321.3303; found 1322.3370 (M+H)⁺.

**Trimer 7c.** Following the general procedure for methyl ester deprotection, a solution of **6c** (83 mg, 0.05 mmol) in THF (1.0 mL) and LiOH·H₂O (13 mg, 0.32 mmol) in water (0.2 mL) were stirred at room temperature overnight. After workup, the residue was precipitated with cool diethyl ether to afford 45 mg (65%) of **7c** as a white solid. Mp (decomp at 222 °C). ¹H NMR (400 MHz, DMSO-*d₆*) *δ*: 1.70 (m, 6H, CH₂CH₂CO), 1.98 (t, *J* = 8.7 Hz, 6H, CH₂CH₂CO), 2.48 (s, 9H, COCH₃), 3.12 (dd, *J* = 14.0, 7.2 Hz, 3H, NHCHCH₂), 3.26 (dd, *J* = 14.0, 7.1 Hz, 3H, NHCHCH₂), 3.45 (s, 2H, NHCOCH₂NH₂), 4.47 (q, *J* = 7.5 Hz, 3H, NHCHCH₂), 7.01 - 7.12 (m, 9H, Ar), 7.15 (t, *J* = 7.6 Hz, 3H, Ar), 7.31 (d, *J* = 8.2 Hz, 3H, Ar), 7.64 - 7.73 (m, 4H, Ar, NHCOCH₂NH₂), 7.82 (d, *J* = 8.2 Hz, 6H, Ar), 8.15 (d, *J* = 7.9 Hz, 3H, NHCHCH₂), 11.56 (br s, 3H, ArNH), 12.45 (br s, 3H, COOH). ¹³C NMR (101 MHz, DMSO-*d₆*) *δ*: 27.0 (COCH₃), 27.8 (NHCHCH₂), 29.7 (CH₂CH₂CO), 30.6 (CH₂CH₂CO), 36.2 (NHCOCH₂NH₂), 53.6 (NHCHCH₂), 57.7 (C(NHCOCH₂NH₂)), 111.9, 119.2, 119.7, 119.9, 121.0, 123.5, 125.8, 127.8, 129.5, 134.4, 137.8, 144.5 (Ar), 165.4 (NHCOCH₂NH₂), 172.2 (CH₂CH₂CO), 173.5 (COOH), 197.4 (COCH₃). HRMS (ESI⁺) m/z: calc for C₆₉H₆₈N₈O₁₃S₃ 1312.4068; found 1313.4141 (M+H)+.

**Trimer 7d.** Following the general procedure, a solution of **6d** (60 mg, 0.04 mmol) in THF (0.7 mL) and LiOH·H₂O (9 mg, 0.22 mmol) in water (0.1 mL) were stirred at room temperature overnight. After workup, the residue was purified by CCTLC in the Chromatotron (DCM:Methanol:acetic acid, 4:1:0.05) to yield 32 mg (64%) of **7d** as a white solid. Mp (decomp at 243 °C). ¹H NMR (500 MHz, DMSO-*d₆*) *δ*: 1.68 - 1.77 (m, 12H, CH₂CH₂CO), 3.00 (br s, 3H, NHCHCH₂), 3.29 (br s, 3H, NHCHCH₂), 3.41 (s, 2H, NHCOCH₂NH₂), 4.28 (br s, 3H, NHCHCH₂), 6.97 (br s, 3H, Ar), 7.08 (t, *J* = 7.5 Hz, 3H, Ar), 7.17 (d, *J* = 8.0 Hz, 6H, Ar), 7.21 - 7.33 (m, 6H, Ar), 7.43 - 7.61 (m, 7H, Ar, NHCOCH₂NH₂), 7.81 (d, J= 8.3 Hz, 2H, NHCHCH₂), 11.28 (br s, 3H, ArNH). ¹³C NMR (126 MHz, DMSO-*d₆*) *δ*: 24.6 (NHCHCH₂), 29.4 (CH₂CH₂CO), 30.4 (CH₂CH₂CO), 55.6 (NHCHCH₂), 111.5, 119.1, 120.3, 120.8, 121.7, 123.0, 123.6, 125.8, 125.9, 126.2, 126.4, 127.9, 128.3, 137.8, 144.4, 171.4 (Ar, CO). HRMS (ESI⁺) m/z: calc for C₆₆H₅₉F₉N₈O₁₀S₃ 1390.3373; found 1391.3432 (M+H)⁺.

**Trimer 7e.** Following the general procedure for methyl ester deprotection, a solution of **6e** (63 mg, 0.04 mmol) in THF (0.8 mL) and LiOH·H₂O (10 mg, 0.25 mmol) in water (0.1 mL) were stirred at room temperature overnight. After workup, the residue was purified by CCTLC in the Chromatotron (DCM:MeOH:acetic acid, 4:1:0.05) to yield 25 mg (50%) of **7e** as a white solid. Mp (decomp at 205 °C). ¹H NMR (500 MHz, DMSO-*d₆*) *δ*: 1.70 (m, 6H, CH₂CH₂CO), 1.97 (m, 6H, CH₂CH₂CO), 3.09 (dd, *J* = 14.1, 7.5 Hz, 3H, NHCHCH₂), 3.24 (dd, *J* = 14.0, 6.7 Hz, 3H, NHCHCH₂), 3.42 (s, 2H, NHCOCH₂NH₂), 4.43 (q, *J* = 7.4 Hz, 3H, NHCHCH₂), 7.05 (t, *J* = 7.5 Hz, 3H, Ar), 7.11 (d, *J* = 8.3 Hz, 6H, Ar), 7.15 (t, *J* = 7.8 Hz, 3H, Ar), 7.31 (d, *J* = 8.2 Hz, 3H, Ar), 7.62 (br s, 1H, NHCOCH₂NH₂), 7.67 (d, *J* = 8.2 Hz, 6H, Ar), 7.72 (d, *J* = 8.1 Hz, 3H, Ar), 8.02 (d, *J* = 8.2 Hz, 3H, NHCHCH₂), 11.56 (br s, 3H, ArNH). ¹³C NMR (126 MHz, DMSO-*d₆*) δ: 27.8 (NHCHCH₂), 29.8 (CH₂CH₂CO), 30.4 (CH₂CH₂CO), 40.6 (NHCOCH₂NH₂), 54.0 (NHCHCH₂), 57.9 (C(NHCOCH₂NH₂)), 108.1, 111.9, 119.2, 119.7, 119.9, 120.0, 120.1, 123.6, 126.4, 127.8, 133.2, 137.8, 145.4 (Ar), 165.8 (NHCOCH₂NH₂), 172.1 (CH₂CH₂CO), 173.6 (COOH). HRMS (ESI⁺) m/z: calc for C₆₆H₅₉N₁₁O₁₀S₃ 1261.3608; found 1262.3670 (M+H)⁺.

**Trimer 7f.** Following the general procedure for methyl ester deprotection, a solution of **6f** (70 mg, 0.05 mmol) in THF (1.1 mL) and LiOH·H₂O (14 mg, 0.33 mmol) in water (0.1 mL) were stirred at room temperature overnight. After workup, the residue was purified by CCTLC in the Chromatotron (DCM:Methanol:acetic acid, 3:1:0.05) to yield 27 mg (40%) of **7f** as a white solid. Mp (decomp at 191 °C). ¹H NMR (500 MHz, DMSO-*d₆*) *δ*: 1.67 (m, 6H, CH₂CH₂CO), 1.30 (m, 6H, CH₂CH₂CO), 2.89 (m, 3H, NHCHCH₂), 3.07 (dd, *J* = 13.8, 7.8 Hz, 3H, NHCHCH₂), 3.29 (s, 2H, NHCOCH₂NH₂), 4.37 (q, *J* = 7.5 Hz, 3H, NHCHCH₂), 6.98 (t, *J* = 7.6 Hz, 3H, Ar), 7.04 - 7.18 (m, 15H), 7.24 (d, *J* = 8.1 Hz, 3H, Ar), 7.53 (br s, 1H, NHCOCH₂NH₂), 7.69 (m, 3H, Ar), 7.73 (d, *J* = 8.1 Hz, 3H, Ar), 11.33 (br s, 3H, ArNH). ¹³C NMR (126 MHz, DMSO-*d₆*) *δ*: 28.3 (NHCHCH₂), 30.2 (CH₂CH₂CO), 55.2 (NHCHCH₂), 58.0 (C(NHCOCH₂NH₂)), 111.5, 116.5, 116.7, 119.3, 119.5, 120.3, 123.0, 123.1, 128.0, 129.6 (d, *J* = 8.0 Hz), 133.0 (d, *J* = 3.0 Hz), 137.6, 161.1 (d, *J* = 242.9 Hz) (Ar), 171.9 (NHCOCH₂NH₂), 172.6 (CH₂CH₂CO), 174.5 (COOH). HRMS (ESI⁺) m/z: calc for C₆₃H₅₉F₃N₈O₁₀S₃ 1240.3468; found 1241.3568 (M+H)⁺.

### 1.2 Synthesis of trimers of formula (I) wherein R¹ is -H, R² is -SPh-4-NO₂ and R³ is: -NHCOCH₂NHCO(CH₂)_{q}CH₃ (q= 4,6,8,10) in said formula

Acyl chains of different length (4, 6, 8 or 10 methylenes) were introduced as substituents of the NH₂-group of the glycine moiety at the focal point (Scheme 3). Selective NHFmoc deprotection of glycine intermediate 6a with piperidine, followed by coupling of the amino derivative thus formed **(8)** with the corresponding acyl chloride gave **9a-d.** Methyl ester deprotection of **9a-d** (LiOH·H₂O) gave the final compounds **10a-d.**

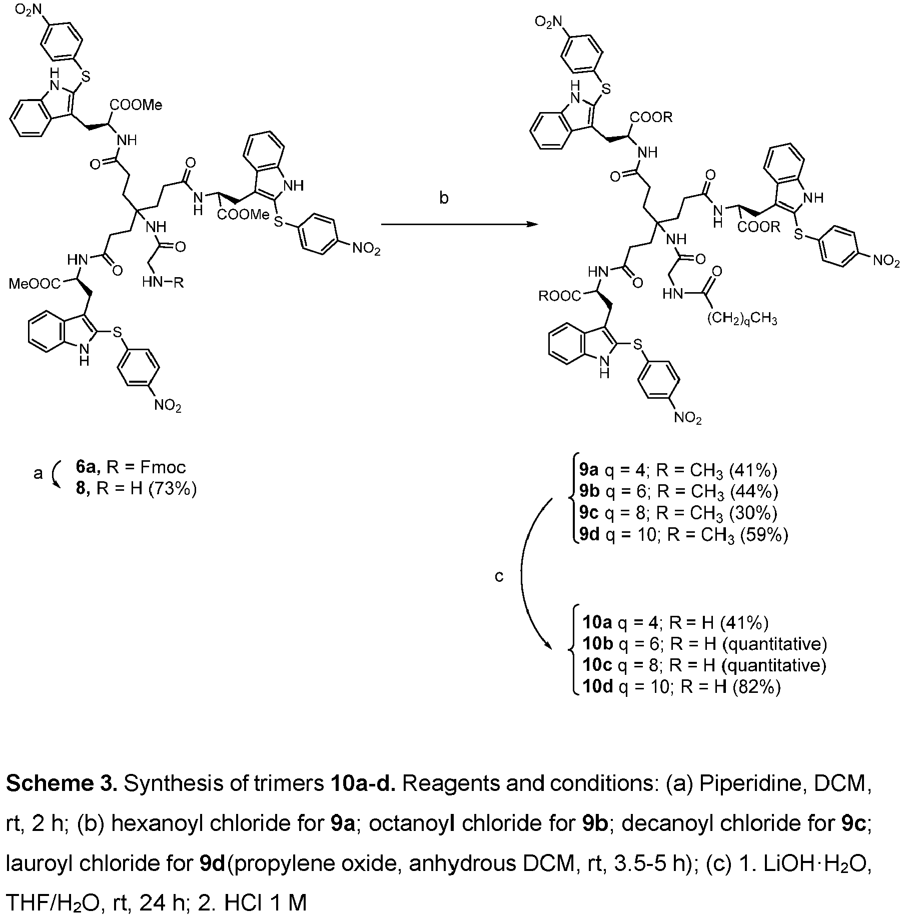

**Trimer 8.** Following the general procedure for selective Fmoc deprotection described for compounds **4a-f,** the Fmoc glycine derivative **6a** (282 mg, 0.18 mmol) and piperidine (176 µL, 1.77 mmol) in DCM (4.0 mL) reacted for 2.5 hours. After workup, the residue was subjected to column chromatography (DCM: methanol, 10:1) to yield 177 mg (73%) of **8** as yellow oil. ¹H NMR (400 MHz, DMSO-*d₆*) *δ*: 1.70 (m, 6H, CH₂CH₂CO), 1.97 (t, *J* = 8.5 Hz, 6H, CH₂CH₂CO), 3.10 - 3.18 (m, 5H, NHCHCH₂, NHCOCH₂NH₂), 3.23 (dd, *J* = 14.0, 7.8 Hz, 3H, NHCHCH₂), 3.42 (s, 9H, COOCH₃), 4.47 (q, *J* = 7.4 Hz, 3H, NHCHCH₂), 7.08 (t, *J* = 7.5 Hz, 3H, Ar), 7.14 - 7.23 (m, 9H, Ar), 7.29 (br s, 1H, NHCOCH₂NH₂), 7.35 (d, *J* = 8.2 Hz, 3H, Ar), 7.62 (d, *J* = 8.0 Hz, 3H, Ar), 8.11 (d, *J =* 9.0 Hz, 6H, Ar), 8.33 (d, *J* = 7.6 Hz, 3H, NHCHCH₂), 11.68 (br s, 3H, ArNH). HRMS (ESI⁺) m/z: calc for C₆₆H₆₅N₁₁O₁₆S₃ 1363.3773; found 1364.3830 (M+H).⁺

**Trimer 9a.** To a cooled solution of **8** (79 mg, 0.06 mmol) in anhydrous DCM (0.6 mL), propylene oxide (81 µL, 1.16 mmol) and hexanoyl chloride (12 µL, 0.09 mmol) were added dropwise. The reaction was stirred at rt for 2 hours and then volatiles were removed. The residue was purified by CCTLC in the Chromatotron (DCM:methanol, 20:0.8) to yield 35 mg (41%) of **9a** as yellow oil. ¹H NMR (400 MHz, CDCl₃) δ: 0.89 (t, *J =* 3.5 Hz, 3H, (CH₂)₄CH₃), 1.23 - 1.34 (m, 8H, (CH₂)₄CH₃), 1.59 (m, 6H, CH₂CH₂CO), 1.92 (m, 6H, CH₂CH₂CO), 3.20 (dd, *J* = 14.3, 8.1 Hz, 3H, NHCHCH₂), 3.39 (dd, *J* = 14.2, 5.5 Hz, 3H, NHCHCH₂), 3.63 (m, 2H, NHCOCH₂NH), 3.67 (s, 9H, COOCH₃), 4.85 (q, *J* = 7.0 Hz, 3H, NHCHCH₂), 6.39 (br s, 1H, NHCOCH₂NH), 6.72 (br s, 3H, NHCHCH₂), 7.04 (d, *J* = 8.6 Hz, 6H, Ar), 7.13 (t, *J* = 7.5 Hz, 3H, Ar), 7.22 (t, *J* = 7.5 Hz, 3H, Ar), 7.31 (m, 3H, Ar), 7.60 (d, *J =* 8.0 Hz, 3H, Ar), 7.98 (d, *J =* 8.6 Hz, 6H, Ar), 9.12 (br s, 3H, ArNH).

**Trimer 9b.** To a cooled solution of **8** (60 mg, 0.04 mmol) in anhydrous DCM (0.5 mL), propylene oxide (62 µL, 0.88 mmol) and octanoyl chloride (11 µL, 0.07 mmol) were added dropwise. The reaction was stirred at rt for 3.5 hours and then volatiles were removed. The residue was purified by CCTLC in the Chromatotron (DCM:methanol, 33:1) to yield 29 mg (44%) of **9b** as yellow oil. ¹H NMR (400 MHz, CDCl₃) *δ*: 0.82 - 0.88 (m, 5H, (CH₂)₆CH₃), 1.16 - 1.32 (m, 10H, (CH₂)₆CH₃), 1.50 (m, 3H, CH₂CH₂CO), 1.69 (m, 3H, CH₂CH₂CO), 1.91 (m, 6H, CH₂CH₂CO), 3.20 (dd, *J* = 14.8, 7.4 Hz, 3H, NHCHCH₂), 3.37 (dd, *J* = 14.6, 5.4 Hz, 3H, NHCHCH₂), 3.57 (m, 2H, NHCOCH₂NH), 3.65 (s, 9H, COOCH₃), 4.86 (q, *J* = 7.0 Hz, 3H, NHCHCH₂), 6.34 (br s, 1H, NHCOCH₂NH), 6.76 (br s, 3H, NHCHCH₂), 7.00 (d, *J* = 8.5 Hz, 6H, Ar), 7.11 (t, *J* = 7.5 Hz, 3H, Ar), 7.20 (t, *J* = 7.6 Hz, 3H, Ar), 7.28 (m, 3H, Ar), 7.58 (d, *J* = 8.0 Hz, 3H, Ar), 7.95 (d, *J* = 8.4 Hz, 6H, Ar), 9.38 (br s, 3H, ArNH).

**Trimer 9c.** To a cooled solution of **8** (77 mg, 0.06 mmol) in anhydrous DCM (0.5 mL), propylene oxide (78 µL, 1.12 mmol) and decanoyl chloride (18 µL, 0.09 mmol) were added dropwise. The reaction was stirred at rt for 4 hours and then volatiles were removed. The residue was purified by CCTLC in the Chromatotron (DCM:methanol, 30:1) to yield 25 mg (30%) of **9c** as yellow oil. ¹H NMR (400 MHz, CDCl₃) δ: 0.83 - 0.90 (m, 6H, (CH₂)₈CH₃), 1.19 - 1.27 (m, 13H, (CH₂)₈CH₃), 1.51 (m, 3H, CH₂CH₂CO), 1.70 (m, 3H, CH₂CH₂CO), 1.92 (m, 6H, CH₂CH₂CO), 3.20 (dd, *J* = 14.3, 7.8 Hz, 3H, NHCHCH₂), 3.38 (dd, *J* = 14.3, 5.6 Hz, 3H, NHCHCH₂), 3.62 (m, 2H, NHCOCH₂NH), 3.66 (s, 9H, COOCH₃), 4.85 (td, *J* = 7.9, 5.6 Hz, 3H, NHCHCH₂), 6.25 (br s, 1H, NHCOCH₂NH), 6.63 (br s, 3H, NHCHCH₂), 7.03 (d, *J* = 8.9 Hz, 6H, Ar), 7.12 (t, *J* = 7.5 Hz, 3H, Ar), 7.22 (t, *J* = 7.5 Hz, 3H, Ar), 7.28 (m, 3H, Ar), 7.59 (d, *J* = 8.0 Hz, 3H, Ar), 7.96 (d, *J* = 8.9 Hz, 6H, Ar), 9.22 (br s, 3H, ArNH).

**Trimer 9d.** To a cooled solution of **8** (50 mg, 0.04 mmol) in anhydrous DCM (0.5 mL), propylene oxide (52 µL, 0.74 mmol) and lauroyl chloride (13 µL, 0.05 mmol) were added dropwise. The reaction was stirred at rt for 5 hours and then volatiles were removed. The residue was purified by CCTLC in the Chromatotron (DCM:methanol, 28:1) to yield 34 mg (59%) of **9d** as yellow oil. ¹H NMR (400 MHz, CDCl₃) δ: 0.83 - 0.89 (m, 7H, (CH₂)₁₀CH₃), 1.15 - 1.30 (m, 16H, (CH₂)₁₀CH₃), 1.51 (m, 3H, CH₂CH₂CO), 1.71 (m, 3H, CH₂CH₂CO), 1.92 (m, 6H, CH₂CH₂CO), 3.20 (dd, *J* = 14.3, 7.6 Hz, 3H, NHCHCH₂), 3.38 (dd, *J* = 14.3, 5.4 Hz, 3H, NHCHCH₂), 3.59 (s, 2H, NHCOCH₂NH), 3.66 (s, 9H, COOCH₃), 4.86 (q, *J =* 7.2 Hz, 3H, NHCHCH₂), 6.30 (br s, 1H, NHCOCH₂NH), 6.71 (br s, 3H, NHCHCH₂), 7.01 (d, *J* = 8.6 Hz, 6H, Ar), 7.12 (t, *J* = 7.4 Hz, 3H, Ar, 7.21 (t, *J* = 7.5 Hz, 3H, Ar), 7.29 (m, 3H, Ar), 7.59 (d, *J* = 8.0 Hz, 3H, Ar), 7.96 (d, *J* = 8.5 Hz, 6H, Ar), 9.33 (br s, 3H, ArNH).

**Trimer 10a.** Following the general procedure for methyl ester deprotection described for **7a-f,** a solution of **9a** (35 mg, 0.02 mmol) in THF (0.5 mL) and LiOH·H₂O (6 mg, 0.14 mmol) in water (0.1 mL) were stirred at room temperature overnight. After workup, the residue was precipitated with cool diethyl ether to afford 14 mg (41%) of **10a** as an amorphous yellow solid. ¹H NMR (500 MHz, DMSO-*d₆*) δ: 0.80 (m, 3H, (CH₂)₄CH₃), 1.16 - 1.27 (m, 8H, (CH₂)₄CH₃)), 1.67 (m, 6H, CH₂CH₂CO), 1.96 (m, 6H, CH₂CH₂CO), 3.11 (dd, J = 14.1, 7.2 Hz, 3H, NHCHCH₂), 3.24 (dd, J = 14.0, 6.9 Hz, 3H, NHCHCH₂), 3.62 (d, J = 5.3 Hz, 2H, NHCOCH₂NH), 4.47 (q, J = 7.4 Hz, 3H, NHCHCH₂), 7.06 (ddd, J = 8.1, 5.5, 1.0 Hz, 3H, Ar), 7.12 - 7.21 (m, 9H, Ar), 7.32 (dt, J = 8.1, 0.9 Hz, 3H, Ar), 7.71 (d, J = 8.1 Hz, 3H, Ar), 7.85 (t, J = 5.6 Hz, 1H, NHCOCH₂NH), 8.09 (d, J = 9.1 Hz, 6H), 8.12 (m, 3H, NHCHCH₂), 11.62 (br s, 3H, ArNH), 12.48 (br s, 3H, COOH). ¹³C NMR (126 MHz, DMSO-*d₆*) δ: 14.3 ((CH₂)₄CH₃), 22.3, 25.3 ((CH₂)₄CH₃), 27.7 (NHCHCH₂), 29.5, 29.8, 30.5, 31.4, 35.6, 53.5, 57.1 (CH₂CH₂CO, CH₂CH₂CO, (CH₂)₄CH₃), 112.0, 119.6, 119.81, 120.1, 123.7, 124.7, 126.2, 127.8, 137.9, 145.4, 148.0 (Ar), 168.5, 172.4, 173.0, 173.4 (NHCOCH₂NH, CH₂CH₂CO, NHCOCH₂NHCO, COOH). HRMS (ESI⁺) m/z: calc for C₆₉H₆₉N₁₁O₁₇S₃ 1419.4035; found 1420.4037 (M+H)⁺.

Trimer **10b.** Following the general procedure for methyl ester deprotection described for **7a-f,** a solution of **9b** (29 mg, 0.02 mmol) in THF (0.4 mL) and LiOH·H₂O (5 mg, 0.12 mmol) in water (0.1 mL) were stirred at room temperature overnight. After workup, the residue was precipitated with cool diethyl ether to afford 28 mg (quantitative yield) of **10b** as a yellow solid. Mp (decomp at 154 °C). ¹H NMR (400 MHz, DMSO-*d₆*) *δ*: 0.81 (t, J= 6.8 Hz, 3H, (CH₂)₆CH₃), 1.15-1.27 (m, 8H, (CH₂)₆CH₃), 1.41 - 1.49 (m, 2H, (CH₂)₆CH₃), 1.67 (m, 6H, CH₂CH₂CO), 1.97 (t, *J* = 8.6 Hz, 6H, CH₂CH₂CO), 2.09 (t, *J* = 7.6 Hz, 2H, (CH₂)₆CH₃), 3.12 (dd, *J* = 14.0, 7.2 Hz, 3H, NHCHCH₂), 3.25 (dd, *J* = 14.1, 6.9 Hz, 3H, NHCHCH₂), 3.63 (d, *J* = 5.4 Hz, 2H, NHCOCH₂NH), 4.47 (q, *J* = 7.3 Hz, 3H, NHCHCH₂), 7.07 (t, *J* = 7.5 Hz, 3H, Ar), 7.13 - 7.22 (m, 10H, Ar, NHCOCH₂NH), 7.32 (d, *J* = 8.2 Hz, 3H, Ar), 7.71 (d, *J* = 8.0 Hz, 3H, Ar), 7.85 (m, 1H, NHCOCH₂NH), 8.07 - 8.15 (m, 9H, Ar, NHCHCH₂), 11.62 (br s, 3H, ArNH), 12.49 (br s, 3H, COOH). ¹³C NMR (101 MHz, DMSO-*d₆*) *δ*: 14.4 ((CH₂)₆CH₃), 22.5, 25.7 ((CH₂)₆CH₃), 27.7 (NHCHCH₂), 28.9, 29.2, 29.8, 30.6, 31.6, 35.6, 36.2 (CH₂CH₂CO, CH₂CH₂CO, (CH₂)₃CH₃), 53.5 (NHCHCH₂), 57.2 (C(NHCOCH₂NH₂)), 112.0, 119.6, 119.8, 120.1, 123.7, 124.7, 126.2, 127.8, 137.9, 145.4, 148.0 (Ar), 162.8 (NHCOCH₂NH), 172.4, 173.1 (CH₂CH₂CO, NHCOCH₂NHCO), 173.4 (COOH). HRMS (ESI⁺) m/z: calc for C₇₁H₇₃N₁₁O₁₇S₃ 1447.4348; found 1448.4382 (M+H)⁺.

**Trimer 10c.** Following the general procedure for methyl ester deprotection described for **7a-f,** a solution of **9c** (25 mg, 0.02 mmol) in THF (0.3 mL) and LiOH·H₂O (4 mg, 0.10 mmol) in water (0.1 mL) were stirred at room temperature overnight. After workup, the residue was precipitated with cool diethyl ether to afford 24 mg (quantitative yield) of **10c** as an amorphous yellow solid. ¹H NMR (500 MHz, DMSO-*d₆*) δ: 0.82 (t, *J* = 6.9 Hz, 3H, (CH₂)₈CH₃), 1.16 - 1.20 (m, 10H, (CH₂)₈CH₃), 1.22 - 1.26 (m, 6H, (CH₂)₈CH₃), 1.67 (m, 6H, CH₂CH₂CO), 1.96 (m, 6H, CH₂CH₂CO), 3.11 (dd, *J* = 14.0, 7.2 Hz, 3H, NHCHCH₂), 3.24 (dd, *J* = 14.1, 6.9 Hz, 3H, NHCHCH₂), 3.62 (d, *J* = 5.3 Hz, 2H, NHCOCH₂NH), 4.47 (q, *J* = 7.3 Hz, 3H, NHCHCH₂), 7.06 (t, *J* = 8.0 Hz, 3H. Ar), 7.14 - 7.21 (m, 9H, Ar), 7.32 (d, *J* = 8.1 Hz, 3H, Ar), 7.70 (d, *J* = 8.0 Hz, 3H, Ar), 7.86 (t, *J* = 5.6 Hz, 1H, NHCOCH₂NH), 8.09 (d, *J* = 9.0 Hz, 6H, Ar), 8.13 (d, *J* = 8.0 Hz, 3H, NHCHCH₂), 11.62 (br s, 3H, ArNH), 12.43 (br s, 3H, COOH). ¹³C NMR (126 MHz, DMSO-*d₆*) δ: 14.4 ((CH₂)₈CH₃), 22.5, 25.7 ((CH₂)₈CH₃), 27.7 (NHCHCH₂), 29.1, 29.2, 29.3, 29.4, 29.8, 30.5, 31.7, 34.1, 35.6, 53.4, 57.1 (CH₂CH₂CO, CH₂CH₂CO (CH₂)₈CH₃), 112.0, 119.6, 119.8, 120.1, 123.7, 124.7, 126.2, 127.8, 137.9, 145.4, 148.0 (Ar), 168.5 (NHCOCH₂NH), 172.4, 173.1, 173.4 (CH₂CH₂CO, NHCOCH₂NHCO, COOH), HRMS (ESI⁺) m/z: calc for C₇₃H₇₇N₁₁O₁₇S₃ 1475.4661; found 1476.4729 (M+H)⁺.

**Trimer 10d.** Following the general procedure for methyl ester deprotection described for **7a-f,** a solution of **9d** (34 mg, 0.02 mmol) in THF (0.5 mL) and LiOH·H₂O (6 mg, 0.13 mmol) in water (0.1 mL) were stirred at room temperature overnight. After workup, the residue was precipitated with cool diethyl ether to afford 27 mg (82%) of **10d** as a yellow solid. Mp (decomp at 118 °C). ¹H NMR (400 MHz, DMSO-*d₆*) *δ*:0.82 (t, *J* = 7.1 Hz, 3H, (CH₂)₁₀CH₃), 1.16 - 1.26 (m, 16H, (CH₂)₁₀CH₃), 1.67 (m, 6H, CH₂CH₂CO), 1.97 (m, 6H, CH₂CH₂CO), 2.09 (t, *J* = 7.6 Hz, 2H, (CH₂)₁₀CH₃), 2.18 (t, *J* = 7.4 Hz, 2H, (CH₂)₁₀CH₃), 3.12 (dd, *J* = 14.0, 7.2 Hz, 3H, NHCHCH₂), 3.25 (dd, *J* = 14.1, 6.9 Hz, 3H, NHCHCH₂), 3.62 (d, *J* = 5.4 Hz, 2H, NHCOCH₂NH), 4.47 (q, *J* = 7.3 Hz, 3H, NHCHCH₂), 7.06 (t, *J* = 7.5 Hz, 3H, Ar), 7.13 - 7.23 (m, 10H, Ar, NHCOCH₂NH), 7.32 (d, *J* = 8.1 Hz, 3H, Ar), 7.71 (d, *J* = 8.0 Hz, 3H, Ar), 7.85 (t, *J* = 5.4 Hz, 1H, NHCOCH₂NH), 8.10 (d, *J* = 8.2 Hz, 6H, Ar), 8.12 (m, 3H, NHCHCH₂), 11.62 (br s, 3H, ArNH), 12.45 (br s, 3H, COOH). ¹³C NMR (101 MHz, DMSO-*d₆*) *δ*: 14.4 ((CH₂)₁₀CH₃), 22.6, 25.0, 25.7 ((CH₂)₁₀CH₃), 27.7 (NHCHCH₂), 29.0, 29.1, 29.2, 29.3, 29.4, 29.5, 29.6 (CH₂CH₂CO, CH₂CH₂CO, (CH₂)₁₀CH₃), 31.8, 34.1, 35.6 ((CH₂)₁₀CH₃), 40.7 (NHCOCH₂NH₂), 53.4 (NHCHCH₂), 57.2 (C(NHCOCH₂NH₂)), 112.0, 119.6, 119.8, 120.1, 120.2, 123.7, 124.7, 126.2, 127.8, 137.9, 145.4, 148.0 (Ar), 168.5 (NHCOCH₂NH), 172.4, 173.4 (CH₂CH₂CO, NHCOCH₂NHCO), 175.0 (COOH). HRMS (ESI⁺) m/z: calc for C₇₅H₈₁N₁₁O₁₇S₃ 1503.4974; found 1504.5040 (M+H)⁺.

### Example 2: Synthesis of tetramers of formula (II)

A similar convergent strategy was used for the synthesis of tetramers of formula (II), In this case the extended pentaerythritol derivative 11 (described in: Newkome, G. R.; Weis, C. D. 6,6-Bis(Carboxy-2-Oxabutyl)-4,8-Dioxaundecane-1,11-Dicarboxylic Acid. Org. Prep.Proced. Int. 1996, 28 (2), 242-244. Flores, A.; Camarasa, M. J.; Perez-Perez, M. J.; San-Félix, A.; Balzarini, J.; Quesada, E. Multivalent Agents Containing 1-Substituted 2,3,4-Trihydroxyphenyl Moieties as Novel Synthetic Polyphenols Directed against HIV-1. Org. Biomol. Chem. 2014, 12 (28), 5278-5294) was used as starting material (Scheme 4). Reaction of **11** with intermediate **4a** afforded the OMe protected derivative 12 (50% yield) whose subsequent deprotection (LiOH·H₂O) gave the final compound 13 (89% yield).

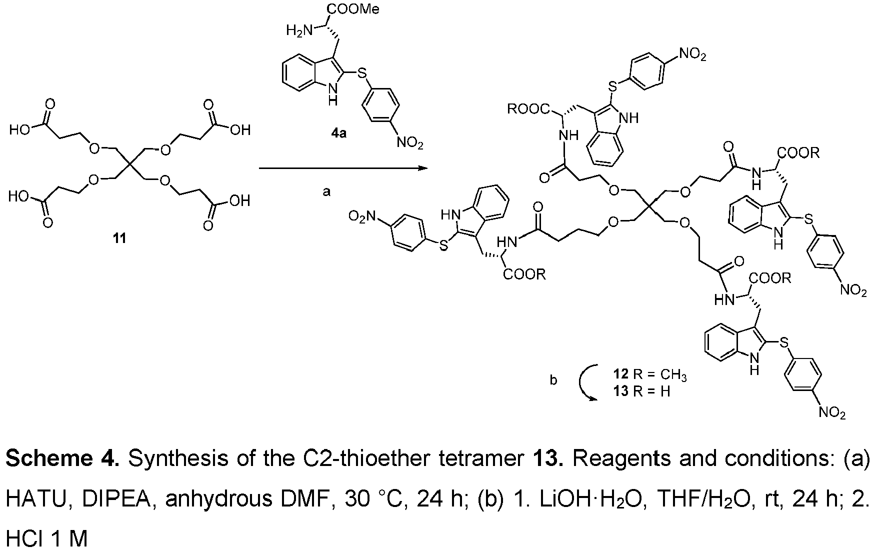

**Tetramer 12.** Following the general coupling procedure described for **6a-f,** tetraacid **8** (100 mg, 0.23 mmol), **4a** (410 mg, 1.10 mmol), HATU (455 mg, 1.20 mmol) and DIPEA (367 µL, 2.12 mmol) in anhydrous DMF (10.0 mL) reacted for 24 hours. After workup, the residue was purified by CCTLC in the Chromatotron (DCM:methanol, 40:1) to yield 216,53 mg (50%) of 12 as a white foam. ¹H NMR (300 MHz, CDCl₃) *δ*: 2.20 (t, *J* = 5.4 Hz, 8H, COCH₂), 2.39 (d, *J* = 9.2 Hz, 4H, OCH₂C), 2.75 (d, *J* = 9.1 Hz, 4H, OCH₂C), 3.04 - 3.46 (m, 16H, Hβ and CH₂O), 3.61 (s, 12H, COOCH₃), 4.91 (m, 4H, Hα), 6.64 (d, *J* = 8.2 Hz, 4H, NHCO), 6.98 (m, 8H, Ar), 7.14 (m, 12H, Ar), 7.57 (m, 4H, Ar), 7.98 (m, 8H, Ar), 9.64 (s, 4H, ArNH).

**Tetramer 13.** Following the general procedure for methyl ester deprotection described for **7a-f,** a solution of **12** (24 mg, 0.03 mmol) in THF (8 mL) and LiOH·H₂O (4.38 mg, 0.24 mmol) in water (2 mL) were stirred at room temperature overnight. After workup, the residue was precipitated with cool diethyl ether to afford 20.7 mg (89%) of **13** as a white foam. ¹H NMR (400 MHz, DMSO-*d₆*) *δ* 2.19 (m, 8H, CH₂O), 3.10 and 3.29 (m, 24H, COCH₂, Hβ and OCH₂), 4.53 (m, 4H, Hα), 7.05 (t, *J* = 7.5 Hz, 4H, Ar), 7.18 (dd, *J* = 8.2, 6.0 Hz, 12H, Ar), 7.33 (d, *J* = 8.2 Hz, 4H, Ar), 7.71 (d, *J* = 8.0 Hz, 4H, Ar), 8.02 - 8.11 (m, 8H, Ar), 8.17 (d, *J* = 8.2 Hz, 4H, NHCO), 11.59 (s, 4H, ArNH). ¹³C NMR (101 MHz, DMSO-*d*₆) *δ* 27.89, 34.85, 36.19, 45.18, 53.51, 67.39, 69.33, 111.97, 119.64, 119.75, 120.07, 120.10, 123.68, 124.66, 125.37, 126.28, 127.79, 128.49, 137.93, 139.63, 145.42, 147.97, 151.92, 170.45, 173.43. HPLC (gradient: CH₃CN/H₂O, 10-100% of CH₃CN in 10 min): 9.350. HRMS (ESI⁺) m/z: calc for C₈₅H₈₀N₁₂O₂₄S₄ 1780.4291; found 1780.4318 (M+H).⁺

### Example 3: Antiviral activity and toxicity using a pseudotyped-VSV assay

Pseudotyped vesicular stomatitis virus (VSV) carrying a codon-optimized S protein of the Wuhan SARS-CoV-2 strain and encoding both Green Fluorescent Protein (GFP) and firefly luciferase was produced and tittered on Vero cells or A549-Ace2-TMPRSS2 cells (InVivoGen, a549-hace2tpsa) as previously described (Ruiz-Rodriguez, P. et al. Evolutionary and Phenotypic Characterization of Spike Mutations in a New SARS-CoV-2 Lineage Reveals Two Variants of Interest. medRxiv 2021, 2021.03.08.21253075.; Gozalbo-Rovira, R. et al. SARS-CoV-2 Antibodies, Serum Inflammatory Biomarkers and Clinical Severity of Hospitalized COVID-19 Patients. J. Clin. Virol. 2020, 131, 104611). For antiviral assays, 1000 focus forming units were premixed with the compounds at the indicated time for 1 hour at 37°C before the addition of the mixture to cells previously plated in a 96-well plate unless otherwise indicated. In all conditions, a mock treatment with the diluent was included (dimethylsulfoxide (DMSO)). After 16 hours, virus infection was quantified by examining GFP fluorescence on a live-cell microscope (Incucyte S3, Sartorius). Finally, toxicity was assessed by adding 20 µL of a 2.4 mg/mL resazurin solution (Sigma-Aldrich, R7017) to each well, incubating for 1 hour at 37°C and reading fluorescence on a Tecan Spark microplate reader with an excitation of 535 nm and emission of 595 nm. All compounds were tested in triplicate and toxicity or antiviral activity derived by comparison to control treated cells. The dose resulting in a 50% reduction of virus produced GFP signal (IC₅₀) was calculated using the drc package in R using a three-parameter log-logistic function (LL3). Results are shown in Table 1.

### Example 4: Antiviral activity using SARS-CoV-2

A SARS-CoV-2 virus carrying the D614G mutation in the S protein was used. The virus was amplified at a multiplicity of infection of 0.001 for 60 hours on Vero-E6 cells. For antiviral assays, sufficient virus to result in 10% of the cells being infected at 24 hours was premixed with the compounds for 1 hour at 37°C. Subsequently, the mixture was used to infect Vero-E6 cells in a 96-well plate for 1 hour at 37°C. Following 24 hours, the cells were fixed in methanol for 30 min, stained with an antibody recognizing the N protein (Genetex GTX635679, 1:2000 dilution), followed by staining with a mouse anti-rabbit IgG-CFL 488 (Santa Cruz Biotechnology sc-516248, 1:1000 dilution). Virus infection was quantified by examining green fluorescence on a live-cell microscope (Incucyte S3, Sartorius) and the dose resulting in a 50% reduction in green fluorescence (IC₅₀) was calculated as indicated above but using a two-parameter log-logistic function (LL2) function, while toxicity was evaluated by examining cell confluence.
The averages of two independent experiments (examples 3 and 4) for each compound are shown in Table 1 and 2, respectively.

**Table 1: Antiviral (IC₅₀) and toxicity (CC₅₀) data using a pseudotyped-VSV assay**

| **Compound** | **IC₅₀** (µM) **Pseudotype VSV (Vero)** | **CC₅₀** (µM) **(Vero)** | **IC₅₀** (µM) **Pseudotype VSV (A549ACE2-TM PRSS2)** | **CC₅₀** (µM) **(A549ACE2-TMPRSS2)** |
|---|---|---|---|---|
| **7a** | 0.23 | >100 | 2.4 | >100 |
| **7b** | 13.01 | >100 | 6.44 | >100 |
| **7c** | 1.4 | >100 | 1.94 | >100 |
| **7d** | 15.10 | >100 | 9.35 | >100 |
| **7e** | 13.88 | >100 | 8.8 | >100 |
| **7f** | 28.45 | >100 | 31 | >100 |
| **10a** | 5.75 | >100 | 8.5 | >100 |
| **10b** | 5.59 | >100 | 4.59 | >100 |
| **10c** | 11.54 | >100 | 8.9 | >100 |
| **10d** | 17.36 | >100 | 16.57 | >100 |
| **13** | 14.53 | >100 | 13.09 | >100 |

| | | | | |
|---|---|---|---|---|
| IC₅₀: 50% inhibitory concentration for virus replication CC₅₀: 50% cytotoxic concentration | | | | |

**Table 2: Antiviral (IC₅₀) and toxicity (CC₅₀) data against SARS-CoV-2.**

| **Compound** | **IC₅₀** (µM) **SARS-CoV-2 (Vero E6)** | **CC₅₀** (µM) **(Vero E6)** |
|---|---|---|
| **7a** | 3.02 | >100 |
| **7c** | 13.3 | >100 |
| **10b** | 23.7 | >100 |

| | | |
|---|---|---|
| IC₅₀: 50% inhibitory concentration for virus replication CC₅₀: 50% cytotoxic concentration | | |

In conclusion, the compounds of the present invention are able to block viral entry at concentrations ranging from 0.23 to 31 µM using VSV-pseudo particles that express the S protein of the Wuhan SARS-CoV-2 strain in their surface in two different cell lines (Vero and A549ACE2-TMPRSS2).

Moreover, the compounds that have been tested have shown antiviral activity against SARS-CoV-2 in the low micromolar range without reaching the CC₅₀ at the highest concentration tested (100 µM).

## Claims

1. A compound of formula (I) or (II): 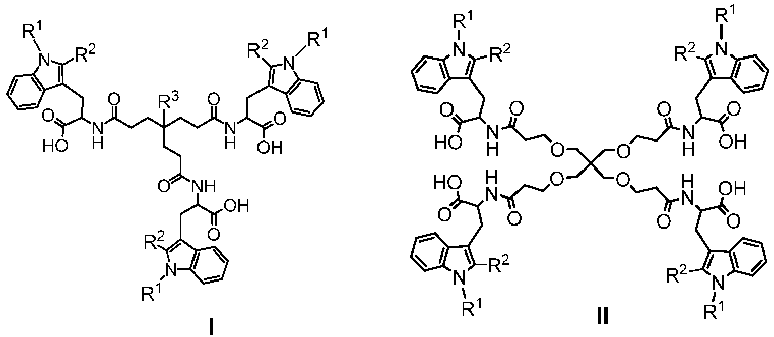 a pharmaceutically acceptable salt, isomers or a solvate thereof, wherein:
R¹ is -H, -CH₃ or -(CH₂)ₙPh, wherein n = 1 or 2 and Ph is a phenyl group which is unsubstituted or substituted by at least one radical selected from the list consisting of: halogen, NO₂ and CF₃,
R² is -SPh, wherein Ph is a phenyl group which is unsubstituted or substituted by at least one radical selected from the list consisting of: halogen, -CF₃, -NO₂, -CN, -NH-SO₂CH₃, -SO₂NH₂, -COCH₃ and -SO₃H,
R³ is -CH₃, -NH₂ or-NHCOWwherein W is -(CH₂)ₚCH₃, -CH₂(OCH₂CH₂)ₚOCH₃, being p an integer between 2 and 10, or -CH(Y)NHZ, wherein
Z is -H, -COCH₃ or -CO(CH₂)_{q}CH₃, being q an integer between 4 and 10 and
Y is a group selected from the list consisting of: H, C₁-C₄ alkyl group unsubstituted or substituted by one radical selected from: phenyl, hydroxyphenyl, -COOH, - CONH₂, -SH, -SCH₃, -OH, -NH₂, 1H-imidazol-4-yl, -CH₂-(1H-indol-3-yl) and 2-pyrrolidinyl.

2. The compound, according to claim 1, wherein R¹ is -H.

3. The compound, according to claim 1 or 2, wherein R² is -SPh, wherein Ph is a phenyl group which is unsubstituted or substituted by one radical selected from the list consisting of: halogen, -CF₃, -NO₂, -CN, -NH-SO₂CH₃, -SO₂NH₂, -COCH₃, -SO₃H.

4. The compound, according to claim 3, wherein R² is -SPh, wherein Ph is a phenyl group which is substituted by one radical selected from the list consisting of: halogen, -CF₃, -NO₂, -CN, -NH-SO₂CH₃, -SO₂NH₂, -COCH₃, -SO₃H, in position *para* with respect to the S atom bonded to the Ph group.

5. The compound, according to any of the previous claims, wherein the phenyl group in R² is substituted by -NO₂ or -COCH₃.

6. The compound, according to any of the previous claims, wherein the compound is a compound of formula (I) wherein R³ is -NHCOW.

7. The compound, according to claim 6, wherein R³ is -NHCOCH(Y)NHZ.

8. The compound, according to claim 7, wherein Y is a group selected from the list consisting of: -H, -CH₃, -CH₂Ph, -CH₂Ph-4-OH, -CH₂COOH, -CH₂CH₂COOH, - CH₂CONH₂, -CH₂CH₂CONH₂, -CH₂SH, -CH₂OH, -CH₂-(1H-imidazol-4-yl), - CH₂CH(CH₃)₂, -CH(CH₃)CH₂CH₃, -(CH₂)₄NH₂, -CH(CH₃)OH, -CH₂-(1H-indol-3-yl), - (CH₂)₂SCH₃, -CH₂-(2-pyrrolidinyl) and -CH₂CH(CH₃)₂.

9. The compound, according to claim 7, wherein R³ is -NHCOCH₂NH₂ or - NHCOCH₂NHCO(CH₂)_{q}CH₃.

10. The compound, according to claim 1, wherein the compound is selected from the next ones: 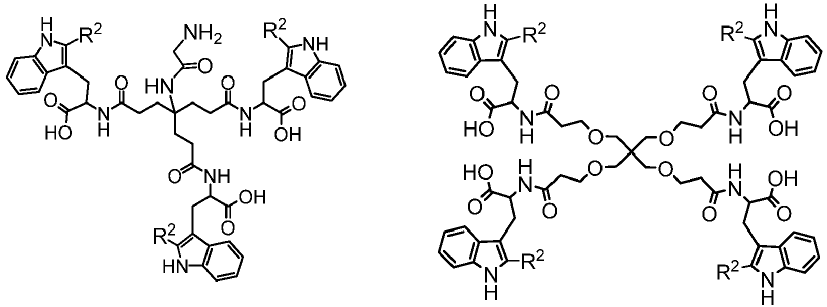 wherein R² is _{:}

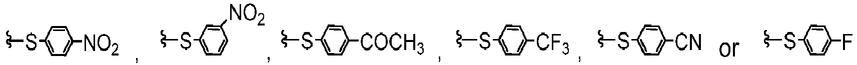

11. The compound, according to claim 1, wherein the compound has the following formula: 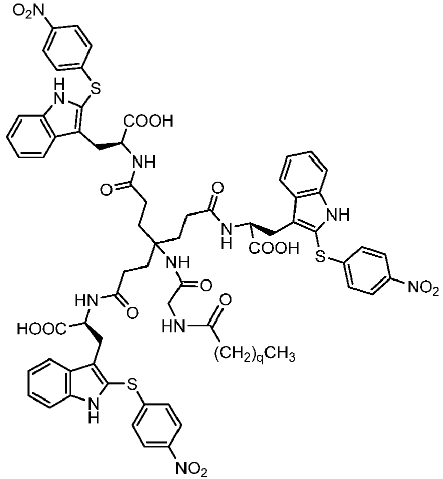 wherein q is 4, 6, 8 or 10.

12. A compound of formula (I) or (II), as defined in any of claims 1-11, for use as a medicament.

13. A compound of formula (I) or (II), as defined in any of claims 1-11, for use in the treatment and/or prevention of betacoronavirus infection.

14. The compound for use, according to claim 13, wherein the betacoronavirus is selected form SARS-CoV, MERS and SARS-CoV-2.

15. A pharmaceutical composition comprising the compound of formula (I) or (II), as defined in any of claims 1-11 and at least one excipient, adjuvant and/or a pharmaceutically acceptable carrier.
